# EUROPEAN PATENT APPLICATION

(11) **EP 1 649 856 A2**
(43) Date of publication of application: **26.04.2006**
(21) Application number: 06001197.0
(22) Date of filing: 09.05.2002
(51) Int. Cl.: A61K 31/436, A61P 1/00, A61P 11/06, A61P 29/00, A61P 37/02

(54) **Use of pimecrolimus for selective immunomodulation**

(30) Priority: 09.05.2001 US 289843 P
(62) Divisional of application: 02725977.9
(71) Applicant: Novartis AG, 4056 Basel (CH)
(72) Inventor: Chibout, Salah-Dine, 68720 Tagolsheim (FR); Cordier, Andre, 4045 Basel (CH); Kehren, Jeanne, 68490 Bantzenheim (FR); Moore, Henrietta Denise, Breitenfurt bei Wien (AT)
(74) Representative: Bourgarel, Denis Jacques Marie

(57) **Abstract**

The invention relates to methods to produce selective immunomodulation in epithelial tissues to treat epithelial inflammatory disorders. This invention provides methods for the use of pimecrolimus to treat epithelial inflammatory disorders including asthma and inflammatory bowel disease while minimizing systemic side effects.

## Description

This application claims priority to U.S. Provisional Application No. 60/289,843 filed May 9, 2001, which is incorporated by reference herein in its entirety.

### Background of the Invention

### Field of the Invention

This invention relates to new methods to produce selective immunomodulation in specific types of tissue for the treatment of diseases in which the inflammation of the tissues that line body cavities plays a prominent role or "epahelial inflammatory disorders" (EID). Examples of such tissues are airway epithelium and GI mucosa. In particular, this invention relates to methods for the use of pimecrolimus (also called ASM981 or ELIDEL™) to treat conditions, such as asthma and inflammatory bowel disease, either as the sole anti-inflammatory agent or as one component of a clinically effective combination of pimecrolimus with inhaled or systemic glucocorticiods or broncodilators in the case of asthma or in combination with glucorticoids, 5-aminosalicylic acid preparations, or immunosuppressants, such as azathioprine (AZA) or cyclosporin A (CyA) in inflammatory bowel disease (IBD). Such combinations having substantially reduced total glucocorticiod or immunosuppressant doses and therefore improved side effect profiles.

### Description of the Related Art

Many diseases involve, as a prominent part of their pathology, inflammation of the epithelial or lining tissues of various organ systems. This includes inflammation of the epithelial tissues that form the skin as well as the closely related epithelial tissues that form the lining of the respiratory tract or the intestinal tract and which may be called the mucosa or mucus membrane. These so-called EIDs are significant for several reasons. Epithelial tissues are often biologically active in a number of ways, including metabolically and as both endocrine organs and immunologic organs and they may play other vital roles, such as in thermoregulation, mucus secretion, gas exchange, control of fluids and electrolytes and nutrient absorption. In addition, it has now also been discovered that the effect of certain drugs on epithelial tissues anywhere in the body, may be significantly different than the effect of that same drug on other regions of the body.

### Asthma

Asthma is an example of a EID and is an extremely common disorder affecting men and women equally; approximately 5% of the adult population of the United States has signs and symptoms consistent with a diagnosis of asthma. Although most cases begin before the age of 25 years, asthma may develop at any time throughout life.

The worldwide prevalence of asthma has increased more than 30% since the late 1970s. The greatest increases in asthma prevalence have occurred in countries that have recently adopted an "industrialized" lifestyle. In addition, the burden of severe asthma has fallen disproportionately on socioeconomically disadvantaged dwellers in the inner city. The reasons for the overall increase in asthma prevalence or the disproportionate fraction of cases in the inner city are not known. Asthma is now among the most common reasons to seek medical treatment. In the United States alone asthma is responsible for about 15 million annual outpatient visits to physicians and for nearly 2 million annual inpatient hospital days of treatment. The yearly direct and indirect costs of asthma care are more than $6 billion dollars, with more than 80% of these costs attributable to direct expenditures on medical care encounters or asthma medications.

The precise etiology of asthma is unknown, however the clinical syndrome is known to be characterized by three distinct components: (1) recurrent episodes of airway obstruction that resolve spontaneously or as a result of treatment; (2) an exaggerated bronchoconstrictor response to stimuli that have little or no effect in nonasthmatic subjects, a phenomenon known as airway hyper-responsiveness; and (3) inflammation of the airways as defined by a variety of criteria.

The pathology of mild asthma, as delineated by bronchoscopic and biopsy studies, is characterized by edema and hyperemia of the mucosa and by infiltration of the mucosa with lymphocytes bearing the T_{H}2 phenotype, mast cells and eosinophils. These cells produce interleukin IL-3, IL-4 and IL-5, and thereby create a microenvironment that promotes the synthesis of immunoglobulin E (IgE), an important allergic effector molecule. Chemokines, such as eotaxin, RANTES, MIP1alpha, and IL-8, produced by epithelial and inflammatory cells, serve to amplify and perpetuate the inflammatory events within the airway.

As a result of these inflammatory insults, the airway wall is thickened by the deposition of type III and type V collagen below the true basement membrane. In more severe chronic asthma, the airway walls thicken as a result of hypertrophy and hyperplasia of airway glands and secretory cells, hyperplasia of airway smooth muscle, and further deposition of submucosal collagen. The shedding of airway epithelium may lead to a denuded airway. These changes occur in a patchy fashion in mild intermittent asthma and become more widespread as the disease becomes more chronic and severe. Morphometric studies of airways from asthmatic subjects have demonstrated airway wall thickening of sufficient magnitude to increase airflow resistance and enhance airway responsiveness. In severe asthma, the airway wall is thickened markedly; in addition, patchy airway occlusion occurs by a mixture of hyperviscous mucus and clusters of shed airway epithelial cells.

An asthma attack results from the episodic airway narrowing that results in obstruction of the airway lumen to airflow. Although it is now well-established that infiltration of the airway with inflammatory cells - especially eosinophils - and mast cells occurs in asthma, the links between these cells and the pathobiologic processes that account for asthmatic airway obstruction have not been clearly delineated. Three possible, but not mutually exclusive, links have been postulated: (1) constriction of airway smooth muscle; (2) thickening of the airway epithelium; and (3) the presence of liquids within the confines of the airway lumen. Among these mechanisms, the constriction of airway smooth muscle due to the local release of bioactive mediators or neurotransmitters is the most widely accepted explanation for the acute reversible airway obstruction in asthma attacks. Several bronchoactive mediators are now thought to be the agents causing airway obstruction in the asthmatic (see Cecil Textbook of Medicine, 21^{st} Ed., Goldman and Bennett, Eds., W.B. Saunders Company, Philadelphia, PA, Chapter 74, pp. 387-393 (2000)).

### Asthma as a predominantly inflammatory illness

The recognition that asthmatic airway narrowing, both at baseline and during disease exacerbation, is due to inflammation has been based on two types of studies in human beings. Increased numbers of inflammatory cells, including eosinophils, macrophages, and lymphocytes, can be found in bronchoalveolar lavage fluid from asthmatic subjects compared to that from normal individuals.

Even asthmatics with normal baseline lung function and no recurrent asthma exacerbations have been found to have increased numbers of eosinophils and other inflammatory cells in their airways. This is true for both allergic and non-allergic asthmatic subjects. After challenge with allergens, there is a further increase in the numbers of inflammatory cells found in the bronchoalveolar lavage fluids in allergic asthmatic subjects.

In addition to bronchoalveolar lavage, lung biopsies have been performed on normal and asthmatic subjects. Compared to normal subjects, asthmatic subjects have increased airway thickness and increased influx of inflammatory cells into the lung tissues. The basis for this inflammation is not entirely clear. Approximately 50% of children, but a much smaller percentage of adults, have clearly defined allergen exposures that can be associated with their asthma. In these patients, exposure to allergens is partially or substantially responsible for their asthmatic inflammation via immediate hypersensitivity-type reactions. Presumably, these reactions can be at a smoldering level, resulting in continuous mild to moderate inflammation but not overt bronchoconstriction.

The large majority of adult asthmatic patients, and approximately 50% of asthmatic children, do not have readily identifiable allergic components to their asthma. However, based on epidemiological studies showing that there is a correlation between increasing IgE levels and prevalence of asthma (see, e.g., Burrows et al., N. Engl. J. Med., Vol. 320, pp. 271-277 (1989)), it is possible that a substantial majority of asthma cases actually have an allergic component that is not readily identified by standard procedures such as skin testing or measuring antigen-specific IgE in blood.

Allergic asthma has been used as a model for the general study of asthma, partly because asthmatic attacks can be elicited at will by exposure to the appropriate allergens. Examination of the mechanisms of allergic asthma offers insight into the rationale for the current therapeutic approaches for asthma in general. Mast cells are activated by exposure to allergen. Allergen-specific IgE is bound to the mast cell via Fc receptors. When allergen comes into contact with the IgE, the mast cell is activated and releases a large number of inflammatory mediators. Mechanisms include the release of granule contents, the synthesis of a variety of lipid membrane-derived molecules, and the production of a number of cytokines following initiation of transcription of their messenger RNAs (mRNAs). In this way, an enormous variety of mediators are thus released, each mediator having more than one potent effect on airway inflammation.

The result of the vasodilation, increased vasopermeability, and increased endothelial adhesiveness to leukocytes is an influx of inflammatory cells from the circulation into the tissues. Lymphocytes, eosinophiis and macrophages predominate. Once these newly recruited cells reach the lung, they release their own mediators which have further inflammatory effects.

Asthmatic inflammation is characterized by bronchial hyperreactivity and therefore differs from the inflammation found in other conditions, such as pneumonia. The chronic results are airway edema, smooth muscle hypertrophy, epithelial shedding and bronchial hyperreactivity to nonspecific stimuli such as strong odors, cold air, pollutants and histamine. Asthmatic airway inflammation may cause increased parasympathetic tone, with resulting bronchial narrowing.

The above scheme predicts that a drug affecting only one mediator is unlikely to be of substantial benefit, simply because there are so many mediators participating. For example, histamine clearly is released during allergic asthmatic reactions (see Murray et al., N. Engl. J. Med., Vol. 315, pp.800-804 (1986)), but antihistamines are of little or no benefit in allergic asthma (see Holgate, Clin. Rev. Allergy, Vol. 12, pp. 65-78 (1994)). One also can predict that drugs that more broadly address asthmatic inflammation could be of greater therapeutic benefit than agents that only address bronchoconstriction *per* se (see Goodman & Gilman, "The Pharmacological Basis of Therapeutics", 9^{th} Ed., McGraw-Hill, NY (1996), especially Chapter 28).

### Treatment of asthma

The treatment of asthma involves the administration of two major classes of drugs, i.e., the bronchodilators and the anti-inflammatory drugs. These drugs may be administered intravenously (i.v.), orally or by topical application of the drugs to the lungs by the inhalation of aerosols. Table 1 below lists the drugs that may be of value in the treatment of asthma.

The bronchodilators used in the treatment of asthma are of several classes and indude beta-adrenergic agonists, anti-cholinergic agents and the two closely related drugs, theophylline and aminophylline. The preferred therapy for bronchoconstriction *per se* is the inhalation of a beta-adrenergic agonist. These agents are effective for immediate relief in acute severe asthma. However, the regular use of beta-adrenergic agonists, as opposed to the more time limited use to provide symptomatic acute control, is much more controversial. Chronic use of the beta-adrenergic agonists may exacerbate bronchial hyper responsiveness and provoke tachyphylaxis possibly by reducing the number of beta-adrenergic receptors by repeated use.

In the past, both anti-cholinergic agents and theophylline or aminophylline have been used to produce bronchial dilation but both classes of drugs are of limited efficacy and have significant side effects.

### Use of anti-inflammatory agents in asthma

In the past, there have been two classes of anti-inflammatory agents available for the treatment of asthma: 1) the glucocorticosteroids and 2) cromolyn sodium (INTAL) and the closely related drug nedocromil sodium (TILADE).

Cromolyn does not have bronchodilatory capability but the drug is able to inhibit antigen-induced bronchospasm as well as the release of histamine and other autacoids from sensitized mast cells. This is thought to be due to the inhibition of pulmonary cell degranulation in response to a variety of stimuli including the interaction between cell-bound IgE and specific antigens. Cromolyn has been used in the U.S. for the treatment of asthma since 1971. However, although cromolyn has proven to be a useful therapeutic for patients with mild to moderate asthma it has not proven to be able to reduce or eliminate the need for systemic glucocorticoids in the treatment of patients with relatively severe asthma.

### The Glucocorticoids

The systemic administration of glucocorticoids has long been employed to treat severe chronic asthma or a severe acute exacerbation of asthma. More recently, the development of aerosol formulations of glucocorticoids has improved the safety of glucocorticoid treatment allowing it to be used for moderate asthma. Several different kinds of glucocorticoid inhalers are now available including those using beclomethasone diproprionate, triamcinolone acetonide and flunisolide.

For treatment of the more severe acute exacerbations or chronic severe asthma it is often necessary to use high doses of glucocorticoids for substantial periods of time. For example, for these conditions, systemic glucocorticoids may be needed at doses of 40-60 mg of prednisone daily for 5 days or more. Glucocorticoids are known to produce severe side effects when substantial doses are taken for extend periods of time. These side effects include; alterations in fluid and electrolyte balance, weight gain, hypertension, increased susceptible to infection, peptic ulcer, myopathy, cataracts, osteoporosis, growth retardation and psychiatric symptoms including psychosis, depression and suicide attempts.

### Table 1. Drugs Used in the Treatment of Asthma

### 1. Anti-inflammatory agents

### A) Glucocorticoids / corticosteroids

(preparations of adrenocortical steroids and their synthetic analogs that could be used in the treatment of asthma or IBD with the nonproprietary name and a selection of trade names)

| | |
|---|---|
| Alclometasone diproplonate (ACLOVATE) | Dexamethasone sodium phosphate (DECADRON PHOSPHATE, HEXAOROL PHOSPHATE) |
| Amcinonide (CYCLOCORT) | Diflorasone diacetate (FLORONE, MAXIFLOR) |
| Beclomethasone diproplonate (BECLOVENT, VANCERIL) | Fludrocortisone acetate (FLORINEF ACETATE) |
| Betamethasone (CELESTONE) | Flunisolide (AEROBID, NASALIDE) |
| Betamethasone benzoate (BENISONE, UTICORT) | Fluocinolone acetonide (FLUONID, SYNALAR) |
| Betamethasone dipropionate (DIPROSONE) | Fluocinonide (LIDEX) |
| Betamethasone sodium phosphate (CELESTONE PHOSPHATE) | Fluorometholone (FLUOR-OP, FML) |
| Betamethasone sodium phosphate and acetate (CELESTONE SOLUSPAN) | Flurandrenolide (CORDRAN) |
| Betamethasone valerate (BETA-VAL, VALISONE) | Halcinonide (HALOG) |
| Budesonide (PULMICORT) | Medrysone (HMS LIZUIFILM) |
| Clobetasol propionate (TEMOVATE) | Methylprednisolone (MEDROL) |
| Clocortolone pivalate (CLODERM) | Methylprednisolone acetate (DEPO-MEDROL, MEDROL ACETATE) |
| Cortisol (hydrocortisone) sodium phosphate (HYDROCORTONE PHOSPHATE) | Methylprednisolone sodium succinate (A-METHAPRED, SOLUMEDROL) |
| Cortisol (hydrocortisone) (CORTEF, HYDROCORTONE) | Mometasone furoate (ELOCON) |
| Cortisol (hydrocortisone) acetate (HYDROCORTONE ACETATE) | Paramethasone acetate (HALDRONE) |
| Cortisol (hydrocortisone) butyrate (LOCOID) | Prednisolone (DELTA-CORTEF) |
| Cortisol (hydrocortisone) cypionate (CORTEF) | Prednisolone acetate (ECONOPRED) |
| Cortisol (hydrocortisone) sodium succinate (A-HYDROCORT, SOLU CORTEF) | Prednisolone sodium phosphate (HYDELTRASOL) |
| Cortisol (hydrocortisone) valerate (WESTCORT) | Prednisolone tebutate (HYDELTRA-T.B.A.) |
| Cortisone acetate (CORTONE ACETATE) | Prednisone (DELTASONE) |
| Desonide (DESOWEN, TRIDEStLON) | Triamcinolone (ARISTOCORT, KENACORT) |
| Desoximetasone (TOPICORT) | Triamcinolone acetonide (KENALOG) |
| Dexamethasone (DECADRON) | Triamcinolone diacetate (ARISTOCORT, KENACORT, DIACETATE) |
| Dexamethasone acetate (DECADRON-LA) | Triamcinolone hexacetonide (ARISTOSPAN) |

### B) Non-Steroidal

| | |
|---|---|
| Cromolyn sodium (INTAL) | Nedocromil sodium (TILADE) |

### 2. Bronchodilators

### A) Beta 2-selective adrenergic agonisiic drugs

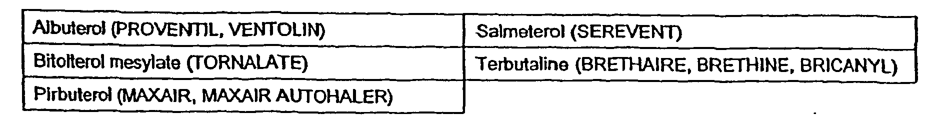

### B) Methylxanfhines

| | |
|---|---|
| I Aminophylline | I Theophylline (THEO-DUR) |

Inhaled glucocorticoids are safer because of the generally lower total systemic dose received. However, even inhaled glucocorticoids have been associated with growth retardation in children, suppression of the hypothalamic-pituitary adrenal axis, bone resorption, changes in carbohydrate and lipid metabolism, cataracts, skin thinning, purpura, dysphasia and candidiasis (see Goodman & Gilman, "The Pharmacological Basis of Therapeutics", 9^{th} Ed., McGraw-Hill, NY (1996), especially Chapters 28 and 59).

The high incidence of side effects in patients who must take systemic or even inhaled glucocorticoids for asthma make it highly desirable that alternate therapies be found that can substitute entirely for, or at least partly replace, glucocorticoids, in the treatment regimens for these patients. This latter is the so-called "steroid-sparing" effect and in patients with severe and/or chronic asthma this reduction in glucocorticoid dosage may prevent or reduce the serious long term adverse consequences of treatment with glucocorticoids.

Accordingly, there is a need for methods of treating asthma which overcome the above limitations in the prior art, and, in particular, which reduce the side effects caused by the administration of high doses of glucocorticoids for long periods of time. In particular, there is a need for methods of treating asthma with drugs with less systemic immunosuppressive effect while still producing a therapeutically effective anti-inflammatory response in the lung. These drugs could be used alone or combined with other drugs known to be effective in the treatment of asthma but would allow the total elimination or significant reduction in dosage of glucocorticoids in the treatment of severe or chronic asthma.

### Inflammatory Bowel Diseases (IBDs)

The IBDs are also examples of EIDs and include ulcerative colitis (UC) and Crohn's disease (CD). These are chronic inflammatory diseases of the gastrointestinal (GI) tract. They are diagnosed by a set of clinical, endoscopic and histologic characteristics, but no single finding is absolutely diagnostic for one disease or the other. Moreover, some patients have a clinical picture that falls between the two diseases and are said to have indeterminate colitis.

The inflammatory response in UC is largely confined to the mucosa and submucosa, but in CD the inflammation starts in the mucosa but in severe cases may extend through the intestinal wall from mucosa to serosa. UC is confined to the colon, and colectomy is a curative procedure. CD, in contrast, can involve any part of the GI tract, although the distal small bowel and the colon are most commonly involved. Resection of the inflamed segment is not curative in CD, and inflammation is likely to recur.

The incidence and prevalence of CD and UC vary with geographic location; the highest rates are for white populations in northern Europe and North America, where the incidence for each disease is about 5 per 100,000 and the prevalence is about 50 per 100,000. Rates in central and southern Europe are lower, and in South America, Asia and Africa lower still. CD and UC are both more common in Jews than non-Jews. In the U.S., the incidence of IBD in the Black population has been one-fifth to one-half that in the White population, but in recent years that gap has narrowed. In northern Europe and North America, the incidence of UC has leveled off but that of CD is still increasing. For both diseases, the incidence is equal in men and women. The peak age at onset is between 1'5 and 25 years of age, with a second, lesser peak between 55 and 65 years of age. Both diseases occur in childhood, although the incidence before 15 years of age is low.

The most important risk factor for IBD is a positive family history. Approximately 15% of IBD patients have affected first-degree relatives, and the incidence among first-degree relatives is 30 to 100 times that of the general population. The best estimates of the lifetime risk of developing IBD among first-degree relatives of affected individuals is 3-9%. The increased incidence among first-degree relatives contrasts to the absence of an increased incidence in spouses of patients. Dizygotic twins have the same rate of concordance as would be expected for siblings, whereas monozygotic twins have higher rates of concordance for both diseases. A susceptibility locus for CD has been mapped to chromosome 16.

In IBD, the lamina propria is infiltrated with lymphocytes, macrophages and other cells of the immune system. An intensive search for the antigens that trigger the immune response has yet to identify a specific microbial pathogen. Anti-colon antibodies of unclear significance have been identified in the sera of UC patients. I BD may also be related to a failure to suppress (or "down-regulate") the normal, finely tuned, low-grade chronic inflammation of the intestinal lamina propria in response to its chronic exposure to luminal antigens.

Whatever the antigenic trigger, activated lamina propria T cells are involved in the pathogenesis of IBD. In CD, the activated lymphocytes appear to be primarily Th1 lymphocytes that produce interferon (IFN)-γ. Pro-inflammatory cytokines Including interleukin (IL)-1 and tumor necrosis factor (TNF)-α amplify the immune response. Intravenous infusion of an antibody to TNF-α is clinically effective in CD. Large numbers of neutrophils enter the inflamed mucosa attracted by chemotactic agents including IL-8 and leukotriene B₄. Epithelial injury in IBD appears to be due to reactive oxygen species from neutrophils and macrophages, as well as to cytokines including TNF-α and IFN-γ.

Mice develop colitis when the genes for IL-2, IL-10 or TGFβ1 are knocked out or when there are certain T-cell receptor mutants, and transgenic rats develop colitis if the human HLA-B27 gene has been introduced. If the same animals are raised in a germ-free environment, colitis does not develop (see Cecil Textbook of Medicine, 21^{st} Ed., Goldman and Bennett, Eds., W.B. Saunders Company, Philadelphia, PA, Chapter 135, pp. 722-729 (2000)).

UC and CD each have characteristic pathologic appearance, but in any given case the pathologic picture may not be specific enough to distinguish between them or to differentiate them from other diseases.

In UC, inflammation begins in the rectum and extends proximally a certain distance and then abruptly stops with a clear demarcation between involved and uninvolved mucosa. Most of the pathologic findings in UC are limited to the mucosa and submucosa. Active UC is marked by neutrophils in the mucosa and submuscoa. There is mucus depletion, mucosal edema and vascular congestion with focal hemorrhage.

### Ulcerative colitis (UC)

The dominant symptom in UC is diarrhea, which is usually associated with blood in the stool (see Table 2), bowel movements are frequent but small in volume as a result of inflamed rectum. Urgency and fecal incontinence may seriously limit the patients ability to function in society. Additional symptoms such as anemia and an elevated leukocyte count and erythrocyte sedimentation rate are useful in confirming severe disease and in followirig the clinical course of a severe exacerbation.

### Crohn's disease (CD)

CD presents with one of three major patterns: 1) disease in ileum and cecum (40% of patients); 2) disease confined to the small intestine (30%); and 3) disease confined to the colon (25%).

The predominant symptoms are diarrhea, abdominal pain and weight loss. Laboratory findings are largely non-specific with anemia and modestly elevated leukocytes.

**Table 2. Criteria for Severity in IBD**

| | |
|---|---|
| Mild | Fewer than four bowel movements per day with little or no blood, no fever and sedimentation rate < 20 mm/hr |
| Moderate | Between mild and severe |
| Severe | Six or more bowel movements per day with blood, fever, anemia and sedimentation rate > 30 mm/hr |

### Drug treatment of IBD

The current pharmacotherapy for IBD can be broadly defined under 5 headings: corticosteroids, aminosalicylates (ASAs), immunosuppressants, antibiotics and cytokine-based therapies.

### Steroids

Corticosteroids are the most commonly used agents for the treatment of moderate to severe IBD. The therapeutic benefit is offset by the well-known side effects attributable to systemic action and suppression of endogenous adrenal function. In UC steroids are used for the acute therapy and are neither effective nor indicated for prevention of relapse. In CD, primarily prednisone and prednisolone are used for moderate to severe rebound effects in a percentage of the patient population, while a further percentage are unresponsive to treatment. Due to the severe side effects of long-term steroid use many experts now felt that alternative therapy to steroids should be sought whenever possible.

### 5-aminosalicylic acid preparations

Sulphasalazine is the most commonly prescribed ASA in the U.S. However, up to 45% of patients and 80% of healthy control subjects report side effects related to the sulphapyridine moiety. Preparations lacking the sulphapyridine have to be delivered topically, such as by suppository or in an enteric coating to delay release. The ASAs are the primary therapies for mild or moderate UC. However, they are not well-tolerated (less than rectal corticosteroids) and have not been evaluated in severe UC. Their main value in a treatment regimen is to prolong the period of remission. In CD, the ASAs fall short of the effectiveness of steroids in active disease and have not shown conclusive evidence of benefit in the quiescent phases.

### Immunosuppression

Azathioprine (AZA) and 6-mercaptopurine inhibit the DNA,synthetic phase of the cell cycle through the action of thioinosinic acid on *de novo* purine ribonucleotides. In UC, they are used to reduce the relapse rate and AZA is recognized as a steroid-sparing agent (see Kirk et al., Br. Med. J., Clin. Res. Ed., Vol. 284, No. 6325, pp. 1291-1292 (1982)). Similarly, in CD, AZA was used for maintaining remission and decreasing steroid consumption but at what cost? Nausea, fever, rash and leukopenia are all common in the first month and 3-15% of patients get pancreatitis or more severe allergic reactions. In addition, there is the risk of dysplasia or colon acer.

### Cyclosporin A

CyA has caused a dramatic and highly debated change in the management of severe UC in the last decade (see Sandbom, Inflammatory Bowel Diseases, Vol. 1, pp. 48-63 (1995)). From 50-80% of patients have benefited from short-term treatment and it has provided an alternative to surgery for the 40% who do not respond to i.v. steroids. In the long term, greater than 40-50% avoided eventual colectomy, especially when maintained on AZA. However, the doses used are high and nephrotoxicity and opportunistic infections are a risk. In CD, CyA is viewed as a "bridge" during the delayed onset of action of AZA. It is not effective as an adjunct to steroids or as remission therapy. Relapse on withdrawal seems common and concurrent AZA treatment necessary.

### Antibiotics

Most of the evidence for the use of antibiotics in IBD is circumstantial. In the limited amount of controlled trials available the antibiotics (metrondiazole, ciprofloxacin) have been shown to be nearly as effective as steroids at inducing remission in patients with acute CD. However, they appear to have little effect on the course of UC.

### Cytokine-targeted therapies

For IBD, antibody therapies have been pursued in the clinic. Efficacy of the chimeric anti-TNF alpha antibody Infliximab (REMICADE™) infusion is well-established in CD patients (moderate to severe) resistant to conventional therapy and those with fistulas. It is also under investigation as a maintenance therapy. Remission is achieved in up to 60% of patients, however up to 60% of all patients develop side effects (e.g., nausea, upper respiratory tract infections, abdominal pain and headache). Sixteen percent of patients suffered serious infusion reactions, the majority occurring after the second infusion, especially in the patient population (13-30%) which develop human anti-chimeric antibodies. Humanized antibodies and/or antibody fragments may be less immunogenic and better tolerated. Trials with the recombinant human IL-10 and IL-11 have shown remission in 40-50% of patients. All such cytokine-targeted therapies must be administered by parenteral injection or infusion and this is a serious disadvantage.

### Combinations

Combinations of the above drugs are now being used for the treatment of IBD, e.g., 5-ASA + antibiotics to treat CD and oral 5-ASA + 5-ASA enema + topical hydrocortisone for the treatment of UC.

**Table 3. Drugs Used in the Treatment of IBD**

| |
|---|
| 5-aminosaiicylic acid preparations (sulphasalazine) |
| Antibiotics (metrondiazole and ciprofloxacin) |
| Corticosteroids (prednisone or any of the glucocorticoids/corticosteroids listed in Table 1) |
| Cydosporin A |
| Cytokine-targeted therapies (Infliximab) |
| immunosuppressants (AZA and 6-mercaptopurine) |

### Side effect profile of current treatments for IBD

The side effect profile of all the drugs used to treat IBD can be severe and the problem for the patient in dealing with these toxic drugs is made far worse by the chronic nature of (BD and the consequent need for long-term use of one, or more commonly, a combination of the drugs.

Steroids (systemic and topical) have numerous side effects including suppression of the hypothalamic-pituitary axis, osteoporosis and psychiatric events. A common steroid used is dexamethasone, however any of the steroids listed in Table 1 could potentially be used in the treatment of IBD. 5-ASAs can cause anorexia, gastric Intolerance, nausea, headaches and renal problems, there is also concern over long-term safety. Immunosuppressed patients, apart from the increased risk of infection, can suffer from hepatotoxicity, impaired kidney function and have an increased risk of tumors. Antibiotics have been associated with convulsions, toxic psychosis, dizziness, depression, rash and phototoxicity. TNF-α inhibition during its relatively short time in the clinic has been linked with serious infection and sepsis resulting in some deaths being associated with this treatment.

To summarize the difficulties and problems associated with the current drug treatment of IBD:
- IBD is chronic disease and is often a severe condition with the need for frequent and/or continuous drug treatment.
- First line and maintenance drugs are available, however, efficacy, as well as better tolerance are needed since presently available medications produce only delays until the next relapse.
- Second line drugs have severe toxicity problems and therefore frequent relapses and high rates of surgery cannot be prevented.
- Most of the knovm compounds in development are biologicals which, as injectables, are not universally suited for therapy of a chronic disease.
- Effective orally-administered drugs are preferred and are better tolerated by patients.

As in the case of patients with asthma, the high incidence of side effects in patients with IBD who must take any of the above drugs, particularly any of the glucocorticoids or the immunosuppressants, make it highly desirable that alternate therapies be found that can substitute entirely for, or at least partly replace, glucocorticoids or the immunosuppressants, in the treatment regimens for these patients. This latter is the so-called "steroid-sparing effect and in patients with severe and/or chronic IBD this reduction in glucocorticoid or immunosuppressant dosage may prevent or reduce the serious long term adverse consequences of treatment with these drugs.

Accordingly, there is a need for methods of treating IBD which overcome the above limitations in the prior art, and, in particular, which reduce the side effects caused by the administration of high doses of glucocorticoids or immunosuppressants for long periods of time. In particular, there is a need for methods of treating IBD with drugs with less systemic immunosuppressive effect while still producing a therapeutically effective anti-inflammatory response in the GI tract. These drugs could be used alone or combined with other drugs known to be effective in the treatment of IBD but would allow the total elimination or significant reduction in dosage of glucocorticoids or immunosuppressants in the treatment of severe or chronic IBD.

Discussion or citation of a reference herein shall not be construed as an admission that such reference is prior art to the present invention.

### Summary of the Invention

The active compound of the composition and methods of this invention is pimecrolimus also known as ASM981 or ELIDEL® (these terms are used interchangeably herein). The preparation of pimecrolimus and pharmaceutical compositions containing pimecrolimus are disclosed in Intemational Publications Nos. WO 01/60345 A2, WO 97/03654, WO 99/01458 and WO 01/90110 A1, U.S. Patents No: 6,197,781 and 6,004,973 and in EP 0 427 680 B1 all of which are hereby incorporated by reference in their entirety and for all purposes.

The use of pimecrolimus in the treatment of EIDs, including but not limited to asthma and IBD provides an improved therapeutic index (as compared to other anti-inflammatory agents) because of the epithelial selective anti-inflammatory action of the drug which concentrates the drug effect on the epithelial tissues of, e.g., the airway epithelium of the lung or the mucosa of the GI tract. This unexpected and previously unknown characteristic of the action of pimecrotimus provides a significantly enhanced margin of safety in the treatment of EIDs and allows for the use of doses of pimecrolimus that provide therapeutically effective drug action while minimizing the overall or general systemic immunosuppressive and side effects. This epithelial tissue selective action of pimecrolimus was previously unrecognized. This unexpected discovery in combination with the results of the gene expression studies done during the clinical pimecrolimus trials in psoriatic patients and the results of trials of pimecrolimus in a mouse model of IBD provide the basis for the present invention.

The action of pimecrolimus is now known to effectively down-regulate numerous genes whose gene products are known to be involved in or causative in the inflammatory process and therefore pimecrolimus should be effective in the treatment of the inflammation of epithelium in various regions of the body including the inflammation of the GI mucosa that characterizes IBD and the inflammation of the airway epithelium and the resulting bronchoconstriction that characterize asthma. The powerful and selective anti-inflammatory action of pimecrolimus can substitute for all or part of the total glucocorticoid dose in a treatment regimen requiring glucocorticoids and therefore producing less overall systemic side effects. Thus, the addition of pimecrolimus can result in fewer and less severe side effects in a treatment regimen.

Thus, this invention provides a method of treating or preventing an EIDs, such as asthma or IBD in a human while minimizing the degree of systemic immunosuppression and consequent side effects, comprising administering to a human in need of treatment or prevention of an EIDs, such as asthma or IBD an amount of pimecrolimus sufficient to produce a significant therapeutic effect in epithelial tissue, including but not limited to, lung airway epithelial tissue or GI mucosa but not enough to produce systemic immunosuppression or adverse side effects. In a preferred embodiment of this invention the degree of systemic immunosuppression may be determined by the IL-2 reporter gene assay described below, for example, the concentration of pimecrolimus required to produce 50% inhibition in the assay (the IC₅₀) is known to be 1.5 nM. This concentration is well below that required to produce clinically significant immunosuppression or adverse side effect. The amount of pimecrolimus administered will vary according to the characteristics of the individual patient however typical doses will be in the range of 10-100 mg/day, preferably 20-80, e.g., 40, 50 or 60 mg/day, preferably administered in equal doses twice daily (b.i.d.). The pimecrolimus may be administered by i.v. infusion, transdermal delivery, inhalation of an aerosol or orally as a tablet, a capsule or a liquid suspension or by rectally by suppository.

In addition, the method may further comprise administering to said human another drug or drugs in combination with pimecrolimus. This other drug or drugs may be one or more of the asthma drugs in Table 1 or may be any drug or drugs known or discovered to be useful in the treatment of asthma or IBD including but not limited to: a glucocorticoid, cromolyn sodium or an alpha-adrenergic agonist or, in the case of IBD may be one or more of the drugs now used in the treatment of IBD, including but not limited to; corticosteroids, 5-ASA acid preparations, antibiotics or immunosuppressants, such as AZA or CyA as shown in Table 3.

In addition, this invention also provides a method of treating EIDs, such as asthma or IBD comprising substituting for some of the glucocorticoids or immunosuppressants required in a treatment regimen an amount of pimecrolimus wherein the amount of the glucocorticoid or immunosuppressant required to be administered, in combination with the pimecrolimus is substantially less than the amount required to be therapeutically effective if administered without the addition of pimecrolimus. Thus providing a therapeutically effective treatment regimen with substantially reduced glucocorticoid or other immunosuppressant mediated systemic short- and long-term side effects.

Furthermore the epithelial selective aspect of pimecrolimus action can be augmented and enhanced by local application. Thus, pimecrolimus can be formulated as an aerosol for administration by inhalation for the treatment of asthma and may be administered as an enema in the form of a solution or suspension (or as a suppository) in the case of the treatment of IBD

### Brief Description of the Figures

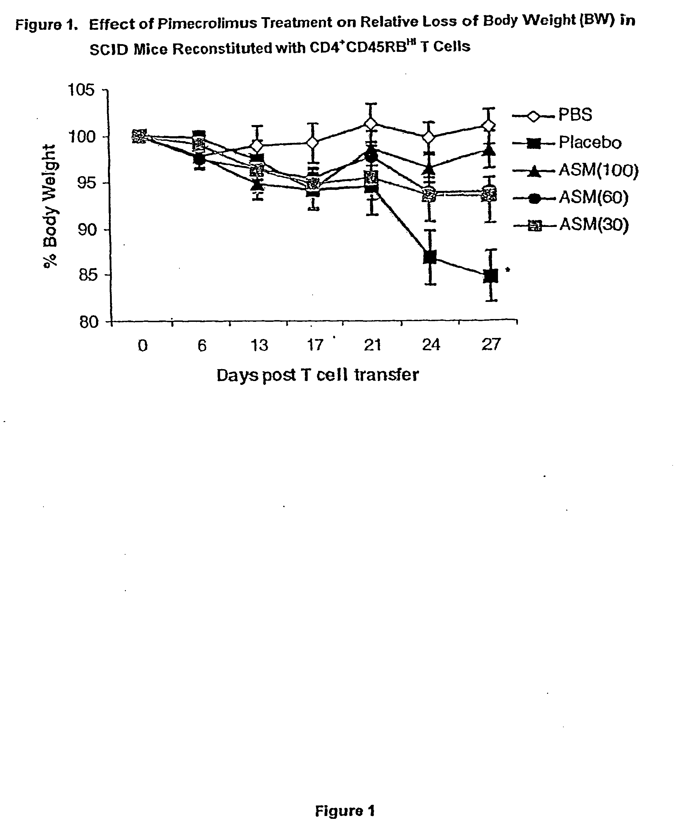

- **Figure 1.**: **Effect of Pimecrolimus Treatment on Relative Loss of Body Weight (BW) in SCID Mice Reconstituted with CD4**^{**+**}**CD45RB**^{**HI**} **T Cells**
SCID mice were reconstituted with 2 x 10⁵ CD4⁺CD45RB^{HI} T cells and oral treatment with pimecrolimus (solid dispersion; dose given in legend in brackets is mg/kg/day) or its placebo started the day after. Treatment was daily. Data are expressed as mean ± s.e.m. % BW relative to that on day 0 for n = 8 mice in each group except the placebo group where n = 7. All values from the non-transferred (PBS) mice were pooled since, in this study, BWs changed similarly independent of the treatment.
*p<0.05 relative to the non-transferred mice.

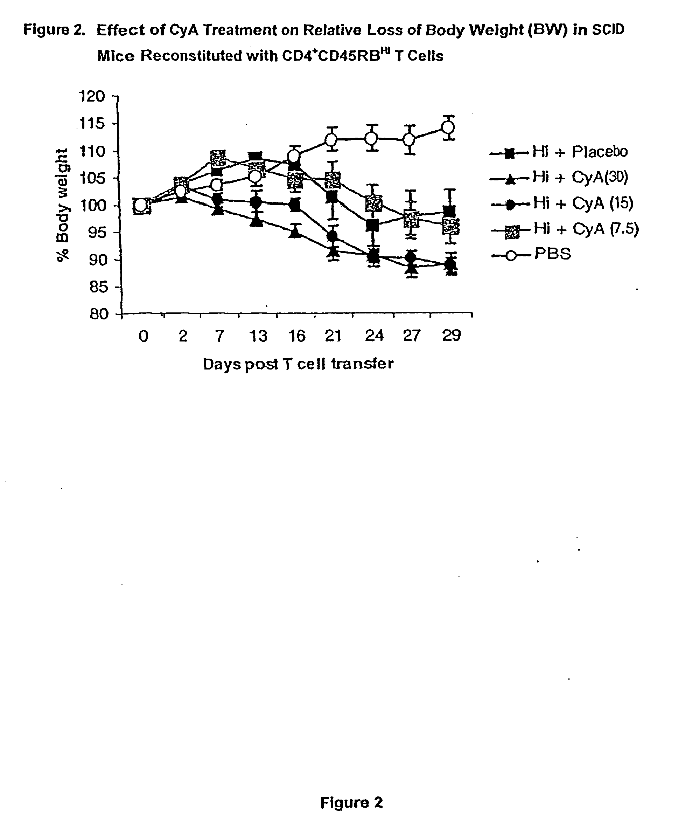

- **Figure 2.**: **Effect of CyA Treatment on Relative Loss of BW in SCID Mice Reconstituted with CD4**^{**+**}**CD45RB**^{**HI**} **T Cells**
SCID mice were reconstituted with 2 x 10⁵ CD4⁺CD45RB^{HI} T cells and osmotic mini-pumps (Alzet) implanted one day later containing CyA given as Sandimmun® (dose given in legend in brackets is mg/kg/day for a 20 g mouse) or its placebo. Data are expressed as mean ± s.e.m. % BW relative to that on day 0 for n = 8 mice in each group except the CyA (30) group where n = 7 since one mouse died shortly after pump implantation. Loss of BW was significant (p<0.05) for all transferred groups relative to the non-transferred (PBS) mice. All values from the non-transferred (PBS) mice were pooled since, in this study, BWs changed similarly independent of the treatment.

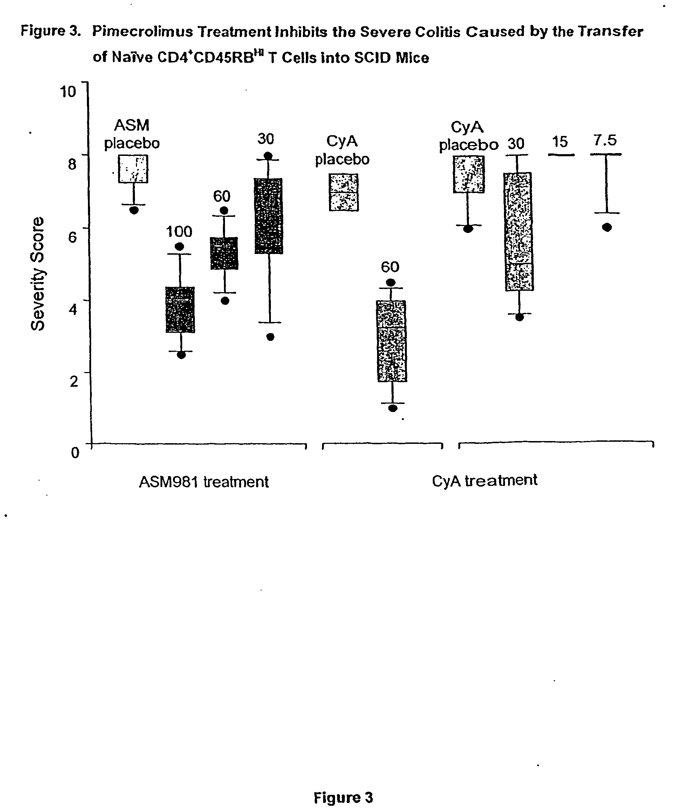

- **Figure 3.**: **Pimecrolimus Treatment Inhibits the Severe Colitis Caused by the Transfer of Naive CD4**^{**+**}**CD45RB**^{**HI**} **T Cells into SCID Mice**
SCID mice were reconstituted with 2 x 10⁵ CD4⁺CD45RB^{HI} T cells and treatment started one day later. Pimecrolimus was given p.o. daily and CyA (given as Sandimmun®) was given via osmotic mini-pump (all doses, indicated on top of the box-plots, are expressed in mg/kg/day). All studies were terminated after 29 days when a piece of colon was washed and fixed in a formalin solution, paraffin embedded, cut into 3 µm sections, stained with H&E and scored on a scale of 0 (no colitis) - 8 (severe). The maximum score was 8 and non-transferred mice scored as 0. Box plots represent the median, average and standard deviation of the individual scores (n = 7 or 8) for each group.

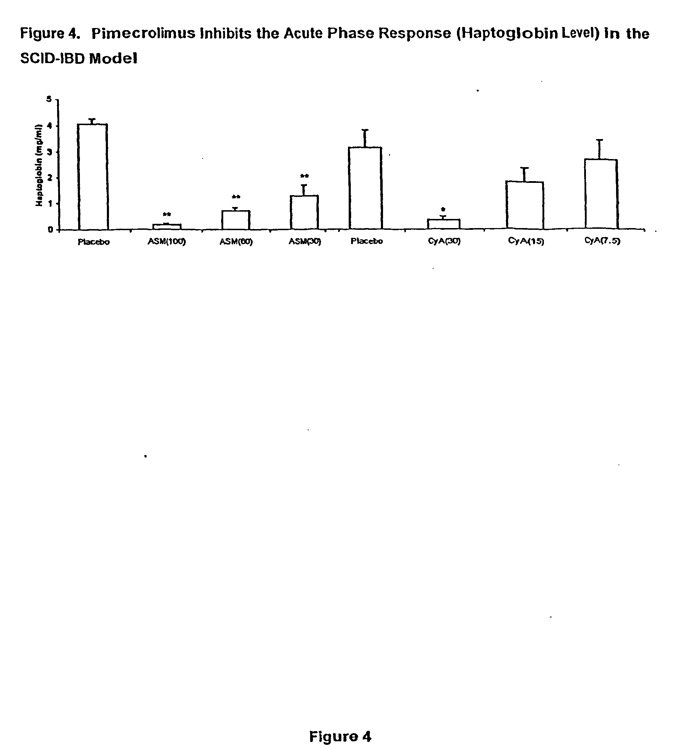

- **Figure 4.**: **Pimecrolimus Inhibits the Acute Phase Response in the SCID-IBD Model**
SCID mice were reconstituted with 2 x 10⁵ CD4⁺CD45RB^{HI} T cells and treatment started one day later. Pimecronmus was given p.o. daily and CyA (given as Sandimmun®) was given via osmotic mini-pump. Both studies were terminated after 29 days when blood was taken and serum levels of haptoglobin, an acute phase response protein in the mouse, were measured. Representative data from two of the SCID-IBD studies are shown above. The data are expressed as mean serum haptoglobin (mg/mL) ± s.e.m. for n = 6 to 8 mice per group.
**p<0.01, *p<0.05 relative to the placebo treated mice in the relevant study.

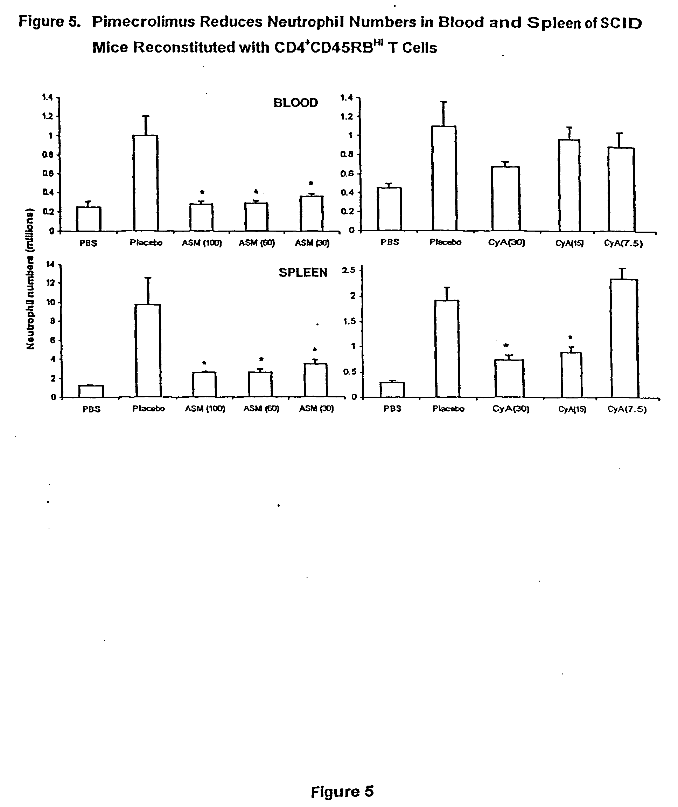

- **Figure 5.**: **Pimecrolimus Reduces Neutrophil Numbers in Blood and Spleen of SCID Mice Reconstituted with CD4**^{**+**}**CD45RB**^{**HI**} **T Cells**
SCID mice were reconstituted with 2 x 10⁵ CD4⁺CD45RB^{HI} T cells and treatment started one day later. Pimecrolimus was given p.o. daily and CyA (given as Sandimmun®) was given via osmotic mini-pump. All studies were terminated after 29 days when blood and spleens were taken, processed into cell suspensions and FACS analyzed for the percentage of neutrophils in each sample. Neutrophils were identified in blood and spleen cell suspensions as cells staining positive for the antibody to Ly-6G (myeloid differentiation antigen; expressed on neutrophils) and also from their forward-, side-scatter characteristics. The numbers of neutrophils were calculated from the total number of cells in each sample and the percentage thereof which were neutrophils. Data are expressed as mean ± s.e.m. neutrophil number for n = 6 to 8 mice in each group.
*p<0.05 calculated relative to the placebo treated animals in the relevant study.

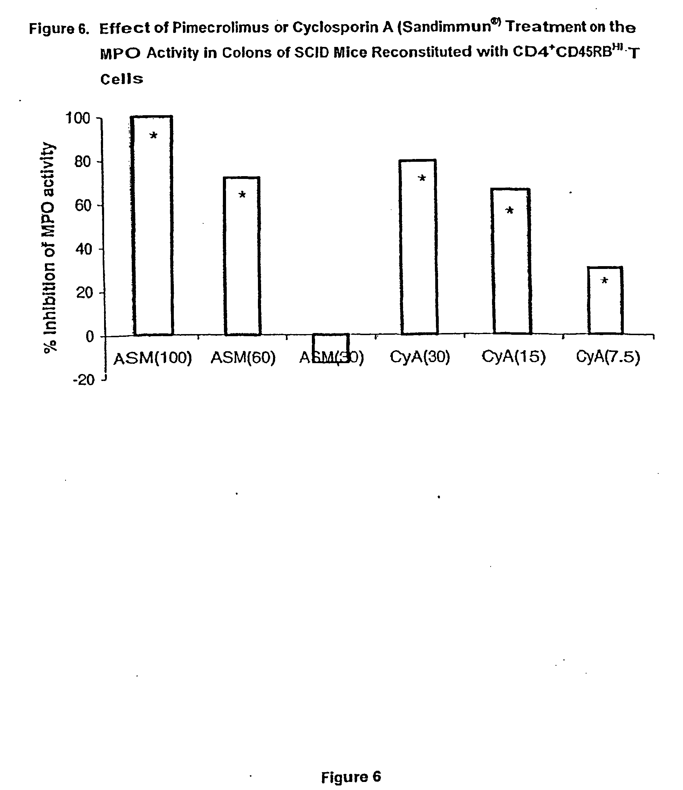

- **Figure 6.**: **Effect of Pimecrolimus or CyA Treatment on the MPO Activity in Colons of SCID Mice Reconstituted with CD4**^{**+**}**CD45RB**^{**HI**} **T Cells**
SCID mice were reconstituted with 2 x 10⁵ CD4⁺CD45RB^{HI} T cells and treatment started one day later. Pimecrolimus was given p.o. daily and CyA (given as Sandimmun®) was given via os motic mini-pump. After 29 days, mice were sacrificed and the colons removed, washed and snap frozen for later preparation and analysis of MPO activity. Each bar (details of numbers of animals in the relevant report) represents the calculated % inhibition of MPO activity (mUnits per µg protein) of the mean activity of between 6 and 8 treated mice relative to 6-8 placebo-treated mice after subtraction of the mean background activity of 6-8 non-transferred mice.
*p<0.05 calculated within each respective study.

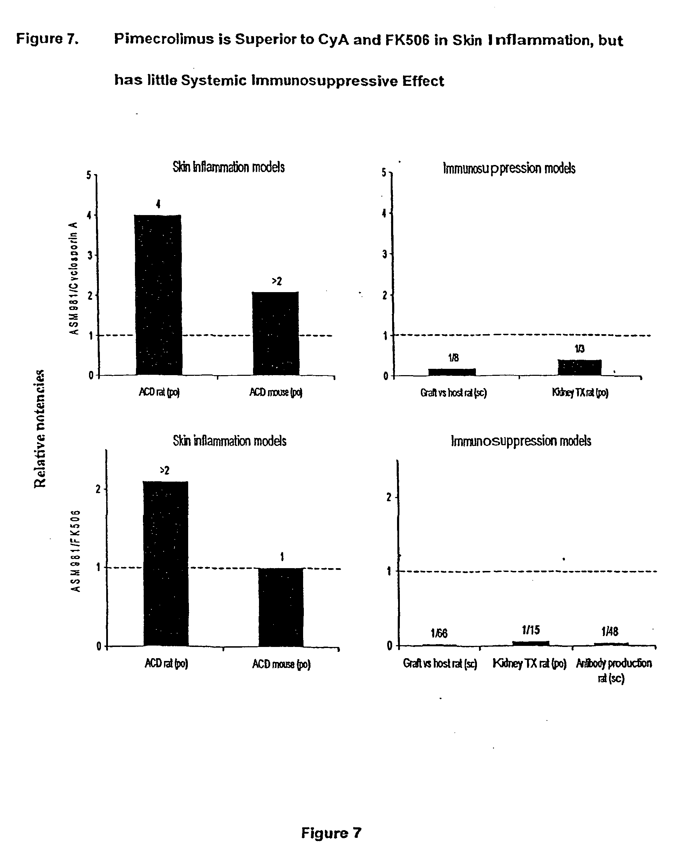

- **Figure 7.**: **Pimecrolimus is Superior to CyA and FK506 in Skin Inflammation, but Has Little Systemic lmmunosuppressive Effect**
(Top left panel) Oral pimecrolimus was slightly greater than 2-fold more efficacious than oral CyA at the dose of 2 x 90 mg/kg in the mouse model of allergic contact dermatitis (ACD) and 4-fold more in the rat ACD model (12.5 mg/kg pimecrolimus similar to 50 mg/kg CyA). Furthermore, at the same doses, oral pimecrolimus was superior or equipotent to FK506 in the rat or mouse ACD model, respectively (bottom left). In contrast to its strong anti-inflammatory properties, pimecrolimus showed minimal immunosuppressive effects compared to either CyA (top right) or FK506 (bottom right) in a series of transplantation and vaccination models.

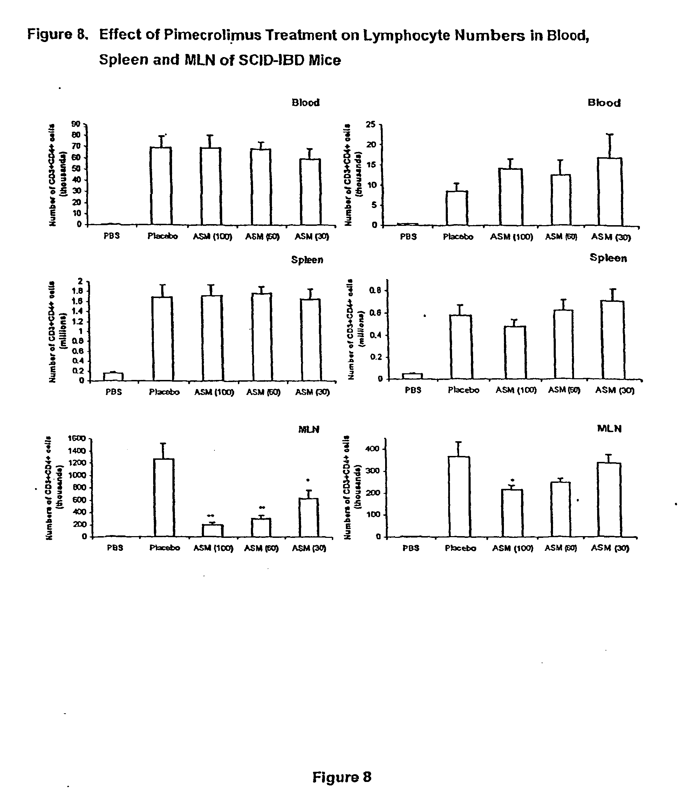

- **Figure 8.**: **Effect of Pimecrolimus Treatment on Lymphocyte Numbers in Blood, Spleen and MLN of CD4**^{**+**}**CD45RB**^{**HI**} **Reconstituted SCID Mice**
SCID mice were reconstituted with 2 x 10⁵ CD4⁺CD45RB^{HI} T cells and treatment started one day later. Pimecrolimus was given p.o. daily. All studies were terminated after 29 days when blood, spleens and MLN were taken, processed into cell suspensions and FACS analyzed for the percentage of lymphocytes in each sample. The total cell number in each sample was found and the number of lymphocytes could therefore be calculated. Data are expressed as mean ± s.e.m. lymphocyte number for n = 6 to 8 mice in each group.
**p<0.01, *p<0.05 calculated relative to the placebo treated animals in the relevant study.

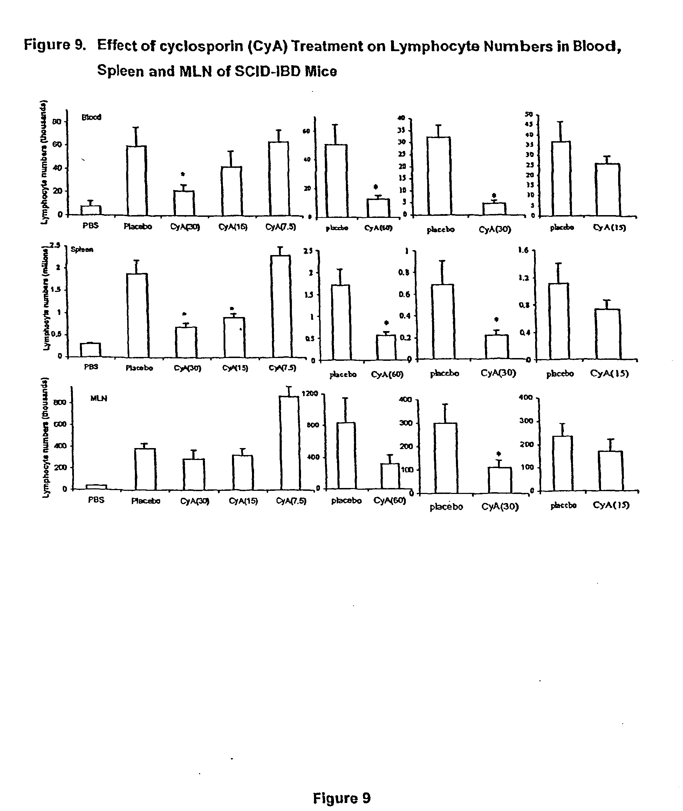

- **Figure 9.**: **Effect of CyA Treatment on Lymphocyte Numbers In Blood, Spleen and MLN of SCID-IBD Mice**
SCID mice were reconstituted with 2 x 10⁵ CD4⁺CD45RB^{HI} T cells and treatment started one day later. CyA was given (given as Sandimmun®) via osmotic mini-pump. All studies were terminated after 29 days when blood, spleens and MLN were taken, processed into cell suspensions and FACS analysed for the percentage of lymphocytes in each sample. The total cell number in each sample was found and the number of lymphocytes could therefore be calculated. Data are expressed as mean ± s.e.m. lymphocyte number for n = 6 to 8 mice in each group.
**p<0.01, *p<0.05 calculated relative to the placebo-treated animals in the relevant study.

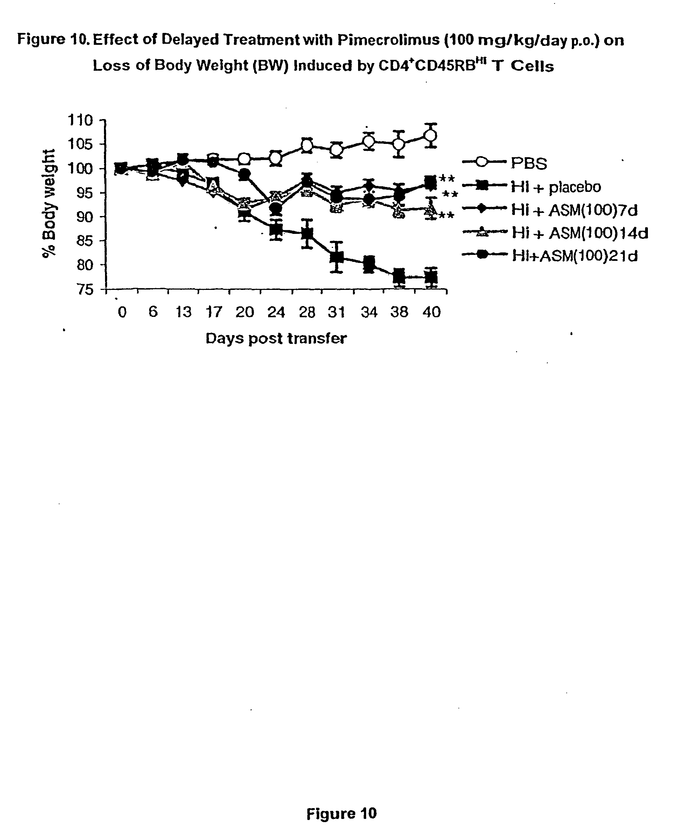

- **Figure 10.**: **Effect of Delayed Treatment with Pimecrolimus (100 mg/kg/day p.o.) on Loss of BW Induced by CD4**^{**+**}**CD45RB**^{**HI**} **T Cells**
SCID mice were reconstituted with 2 x 10⁵ CD4⁺CD45RB^{HI} T cells and treatment with pimecrolimus (100 mg/kg/day p.o.) started after 7, 14 or 21 days and continued daily until 41 days. Treatment with the placebo formulation started after7 days. BW was monitored once a week for the first 2 weeks and then twice weekly. Data are expressed as mean % BW relative to the weight on day 0 ± s.e.m for n = 9 (PBS), n = 8 (ASM(7d) & ASM(21d) and n = 7 (ASM(I4d) and placebo) mice per group. PBS refers to the non-transferred mice which have been pooled with regard to % BW values since no treatment dependent changes were found.
**p<0.01 relative to the placebo treated group at 41 days.

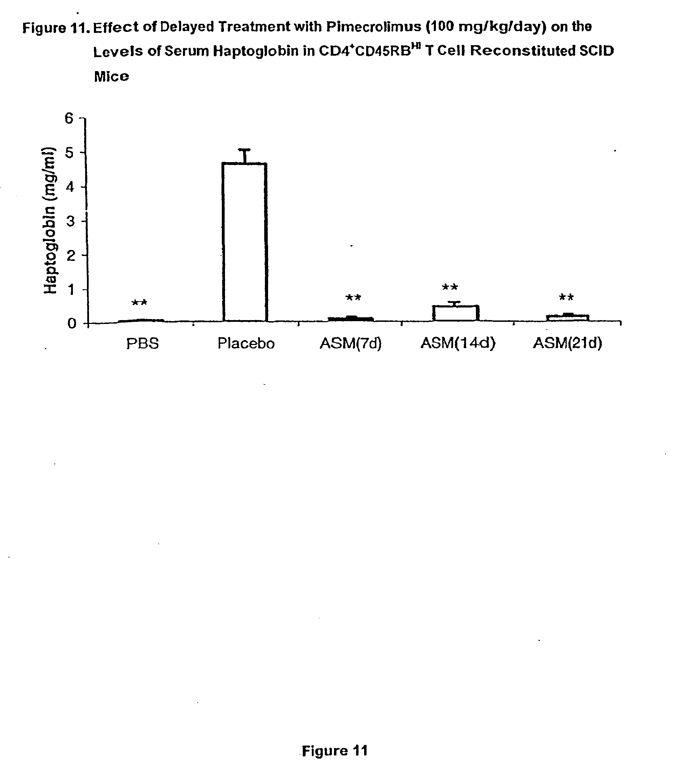

- **Figure 11.**: **Effect of Delayed Treatment with Pimecrolimus (100 mg/kg/day) on the Levels of Serum Haptoglobin in CD4**^{**+**}**CD45RB**^{**HI**} **T Cell Reconstituted SCID** Mice
SCID mice were reconstituted with 2 x 10⁵ CD4⁺CD45RB^{HI} T cells and treatment with pimecrolimus (100 mg/kg/day p.o.) started after 7, 14 or 21 days and continued daily until 41 days. Treatment with the placebo formulation started after 7 days. On day 41, mice were killed and serum kept for haptoglobin assay.
Data are expressed as mean haptoglobin (mg/mL) ± s.e.m for n = 9 (PBS), n = 8 (ASM(7d)) and n = 7 (ASM(I4d), ASM(21 d) and placebo) mice per group. PBS refers to the non-transferred mice which have been pooled with regard to haptoglobin values since no treatment dependent changes were found.
**p<0.01 relative to the placebo treated group at 41 days.

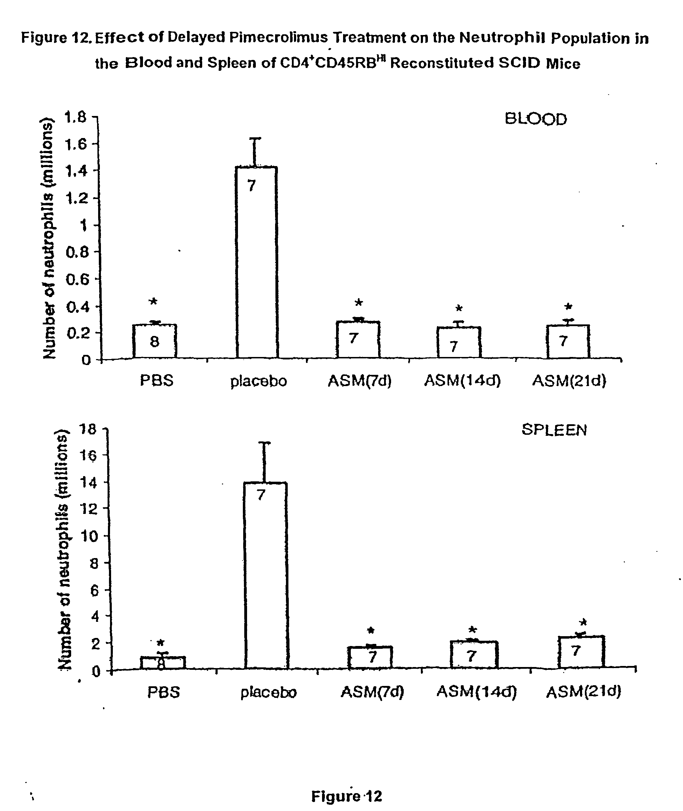

- **Figure 12.**: **Effect of Delayed Pimecrolimus Treatment on the Neutraptril Population in the Blood and Spleen of CD4**^{**+**}**CD45RB**^{**HI**} **Reconstituted SCID Mice**
SCID mice were reconstituted with 2 x 10⁵ CD4⁺CD45RB^{HI} T cells and treatment with pimecrolimus (100 mg/kg/day p.o.) started after7, 14 or 21 days and continued daily until 41 days. Treatment with the placebo formulation started after7 days. On day 41, mice were killed, blood and spleens removed, cell suspensions prepared and incubated with relevant antibodies to identify the neutrophil population by FACS analysis. Data are expressed as the mean neutrophil numbers, calculated from the % positive cells and the total cell number per sample, ± s.e.m for n mice (n in each bar) per group.
*p<0.05 calculated relative to the placebo treated group.

### Detailed Description of the Invention

The present invention encompasses methods for treating or preventing an EID, such as asthma or IBD in a human, while minimizing systemic side effects including but not limited to, opportunistic infections such as those produced by bacterial, viral or fungal pathogens or lymphoproliferative disorders such as lymphomas or other malignancies, which comprises administering to said human a therapeutically effective amount of pimecrolimus. This therapeutically effective amount of pimecrolimus would be sufficient to treat or prevent the EID, such as asthma or IBD but not sufficient to produce significant general systemic immunosuppressant effect or adverse side effects. In one embodiment this dose would an oral dose of between 5 mg/day and 100 mg/day, in a more preferred embodiment the oral dose would be from 20-80 mg/day, and in a most preferred embodiment the oral dose would be between 30 and 60 mg/day in a single or divided dose, preferably in a b.i.d. schedule.

A still further aspect of the present invention includes methods for treating or preventing an EID, such as asthma or IBD in a human while minimizing or reducing systemic side effects, including but not limited to, opportunistic infections, such as those produced by bacterial, viral or fungal pathogens or lymphoproliferative disorders, such as lymphomas or other malignancies, comprising administering to said human a therapeutically effective amount of pimecrolimus. This therapeutically effective amount of pimecrolimus would be sufficient to treat or prevent or to ameliorate at least one symptom of the EID, such as asthma or IBD but not sufficient to produce significant general systemic immunosuppressant effect or adverse side effects, such as opportunistic infections such as those produced by bacterial, viral or fungal pathogens or lymphoproliferative disorders such as lymphomas or other malignancies. The "therapeutically effective amount" of pimecrolimus in the above method is preferably an amount which is effective to treat the EID but which is not systemically immunosuppresive, for example, an amount which does not result in blood levels of pimecrolimus corresponding to an IC₅₀ in an IL-2 reporter gene assay, e.g., as described in U.S. Patent No. 5,897,990, e.g., blood levels of <1.5 ng/mL, preferably <1.0 ng/mL, more preferably 0.5 ng/mL or less. The optimal dose may vary somewhat depending on the individual and the condition to be treated, but suitable total daily oral doses of pimecrolimus for adults for treatment of the above-referenced conditions are typically on the order of 10-100 mg/day, preferably 20-80, most preferably 40-60 mg/day, preferably administered in equal doses b.i.d.

A still further aspect of the present invention includes methods for treating or preventing an EID, such as asthma or IBD in a human while minimizing or reducing systemic side effects, including but not limited to, opportunistic infections, such as those produced by bacterial, viral or fungal pathogens or lymphoproliferative disorders, such as lymphomas or other malignancies, comprising administering to said human a therapeutically effective amount of pimecrolimus. This therapeutically effective amount of pimecrolimus would be sufficient to treat or prevent or to ameliorate at least one symptom of the EID, such as asthma or IBD but not sufficient to produce significant general systemic immunosuppressant effect or adverse side effects. In one embodiment, this dose would an total daily oral dose of pimecrolimus in one single or two or more divided doses that results in an area under the curve (AUC), in a chart of steady-state serum or blood level over time, of less than 500 ng hour/mL over a 24-hour period.

As used herein, the term "AUC" means the area under the curve of the serum or blood level of the drug over time, assessed while the patient is receiving regular, e.g., once a day (q.d.) or b.i.d. or more, doses of the medication. This AUC may be calculated according to the dosing interval, meaning over the time between doses, generally 24, 12, 8 or 6 hours, or it may be calculated over a 24-hour period during steady-state regardless of dosing interval.

A still further aspect of the present invention includes methods for treating or preventing an EID, such as asthma or IBD in a human while minimizing or reducing systemic side effects, including but not limited to, opportunistic infections, such as those produced by bacterial, viral or fungal pathogens or lymphoproliferative disorders, such as lymphomas or other malignancies, comprising administering to said human a therapeutically effective amount of pimecrolimus. This therapeutically effective amount of pimecrolimus would be sufficient to treat or prevent or to ameliorate at least one symptom of the EID, such as asthma or IBD but not sufficient to produce significant general systemic immunosuppressant effect or adverse side effects such as opportunistic infections such as those produced by bacterial, viral or fungal pathogens or lymphoproliferative disorders such as lymphomas or other malignancies. The "therapeutically effective amount" of pimeaolimus in the above method is preferably an amount which is effective to treat the EID but which is not systemically immunosuppresive, for example, an amount which results in minimum trough serum levels above 7 ng/mL but less that 20 ng/mL The optimal dose to produce these trough levels will vary somewhat depending on the individual and the condition to be treated, but suitable total daily oral doses of pimecrolimus for adults for treatment of the above-referenced conditions are typically on the order of 10-100 mglday, preferably 20-80, most preferably 40-60 mg/day, preferably administered in equal doses b.i.d.

A still further aspect of the present invention includes methods for treating or preventing an EID, such as asthma or IBD in a human while minimizing or reducing systemic side effects, including but not limited to, opportunistic infections, such as those produced by bacterial, viral or fungal pathogens or lymphoproliferative disorders, such as lymphomas or other malignancies, comprising administering to said human a therapeutically effective amount of pimecrolimus. This therapeutically effective amount of pimecrolimus would be sufficient to treat or prevent or to ameliorate at least one symptom of the EID, such as asthma or IBD but not sufficient to produce significant general systemic immunosuppressant effect or adverse side effects. The "therapeutically effective amount' of pimecrolimus in the above method is preferably an amount which is effective to treat the EID but which is not systemically immunosuppresive, for example, an amount which results Cₘₐₓ levels of less that 60 ng/mL. The optimal dose to produce these Cₘₐₓ levels will vary somewhat depending on the individual and the condition to be treated, but suitable total daily oral doses of pimecrotimus for adults for treatment of the above-referenced conditions are typically on the order of 10-100 mg/day, preferably 20-80, most preferably 40-60 mg/day, preferably administered in equal doses b.i.d.

As used herein, the term "opportunistic infections produced by bacterial, viral or fungal pathogens" means any infection caused by bacterial, viral or fungal agents that persons with completely intact immune system generally do not get but which do occur in patients with depressed immune system function for any reason. The nature and causative pathogens of these opportunistic infections are well known in the art and include but are not limited to; Herpes simplex virus, varicella-zoster virus, cytomegalovirus, *P. carinii,* S. *pneumoniae, H. influenzae, candida, P. aeruginosa, S. aureus* and *C. difficile.*

As used herein, the term "lymphoproliferative disorders, oHymphomas or other malignancies" means any of the many types of cancer which patients on immunosuppressive therapy are at increased risk for, these include but are not limited to lymphonia of skin or soft tissues, bladder cancer, leukemias, renal or liver tumors (see Cecil Textbook of Medicine, 21^{st} Ed., Goldman and Bennett, Eds., W.B. Saunders Company, Philadelphia, PA, pp. 1045-1046 (2000)).

As used herein, the term "Cₘₐₓ" shall mean the maximum blood level developed after administration of a dose of the drug. The time to reach Cₘₐₓ following administration will depend on the speed of absorption and half-life of the specific drug.

A further aspect of the present invention includes methods for treating or preventing an EID, such as asthma or IBD in a human while reducing or eliminating the need for administration of a glucocorticoid or other immunosuppressant agent. Thus, in this embodiment a combination of pimecrolimus and one or more of the drugs listed in Table 1 in the case of asthma or one or more of the drugs listed in Table 3 for IBD can be used to treat or prevent an EID in a patient in need of such treatment. The dose of pimecrolimus could be determined according to any of the methods disclosed herein. The dose(s) of the glucocorticoid or other immunosuppressant could be reduced while maintaining an equal or better therapeutic effect with the combination.

One of skill in the art could determine the correct reduction in the dose(s) of glucocorticoid or other immunosuppressant agent by assessment of the patients clinical status. In producing this combination treatment the glucocorticoid or other immunosuppressant agent may be started first and then partly replaced by pimecrolimus or the pimecrolimus may be started first and a glucocorticoid or other immunosuppressant agent could then be added to form the combination therapy. In another embodiment the components of the combination therapy could be started at the same time and dose adjustments made based on clinical assessment of the patient

The use of such a combination would allow effective treatment of the EID but because of the decreased need for glucocorticoid or other immunosuppressant agents the overall combination would have reduced systemic immunosuppressant effect and reduced likelihood to produce adverse side effects, including but not limited to, opportunistic infections, such as those produced by bacterial, viral or fungal pathogens or lymphoproliferative disorders, such as lymphomas or other malignancies.

A further aspect of the present invention indudes methods for treating or preventing an EID, such as asthma or IBD in a human while minimizing or reducing systemic side effects, including but not limited to, opportunistic infections, such as those produced by bacterial, viral or fungal pathogens or lymphoproliferative disorders, such as lymphomas or other malignancies, comprising administering an amount of pimecrolimus sufficient to ameliorate at least one symptom of the EID but which produces less systemic immunosuppression than an equally effective dose of a corticosteroid or other immunosupressant drug. Therefore, this method takes advantage of the "therapeutic window" effect of pimecrolimus which occurs because of the enhanced action of pimecrolimus in epithelial tissues of the body. Because of this effect, it is possible to find a dose of pimecrolimus that is therapeutically effective in the region of epithelium Involved in the EID so as to ameliorate, reduce or eliminate one or more of the symptoms of the EID but which will produce less systemic immunosuppressive effect than a dose of corticosteroid or an immunosuppressant, such as CyA or tacrolimus (FK506), powerful enough to produce an equivalent therapeutic effect in the epithelial target tissue.

In this specification, the comparison of the immunosuppressive effect of specific doses of a corticosteroid to the immunosuppressive effect of pimecrolimus refers to a comparison between orally active corticosteroid and oral doses of pimecrolimus, since the local effects of inhaled or other topically active corticosteroids may be very different from the effects of the orally active versions of this class of drugs.

Specifically this method entails treating a patient with a dose of pimecrolimus which is. sufficient to ameliorate at least one symptom of the EID or to reduce or eliminate all or most symptoms of the disorder but which produces less systemic immunosuppression than a dose of a steroid or other immunosuppressant drug sufficient to produce an equal therapeutic effect on the symptoms of the EID. In a preferred embodiment, the degree of systemic immunosuppression produced by the alternative therapies would be measured, e.g., by the IL-2 reporter gene assay described below.

In one embodiment of this method, a patient with a EID, such as asthma or IBD is treated with pimecrolimus at a dose level sufficient to ameliorate at least one symptom of the EID. The systemic immunosuppressant effect of this dose is assayed by use of the IL-2 reporter assay described below. This systemic immunosuppressant effect is compared to that produced by a dose of corticosteroid or other immunosuppressant required to produce an equivalent amelioration of the at least one symptom. The dose of pimecrolimus is chosen to produce a systemic immunosuppressant effect that is less than that produced by the corticosteroid or other immunosuppressant dose required to produce an equivalent effect on the symptoms of the EID.

In another embodiment, a patient who is on a maintenance dose of corticosteroids and/or immunosuppressants for the treatment of an EID, such as asthma or IBD, is treated with pimecrolimus at a dose level sufficient to maintain control of the symptoms of the disease but allow the dose of corticosteroids and/or immunosuppressants to be reduced so that the total systemic immunosuppressant effect of the combination of pimecrolimus and corticosteroids and/or immunosuppressant, as measured, e.g., by the use of the IL-2 reporter assay described below, is less than that produced by the prior higher dose of corticosteroids and/or immunosuppressants alone. In this method the selective action of pimecrolimus on epithelial tissue is employed to produce a "steroid sparing effect" with regard to the treatment regimen as a whole and therefore result less systemic immunosuppression and therefore in fewer and less severe side effects to the patient.

As used herein, the term "EID" means any disease in which the inflammation of the tissues that line one or more body cavities plays a prominent role, examples of such tissues include, but are not limited to, epithelium of skin or respiratory tract and mucosa or mucus membrane of the GI tract.

As used herein, the term "systemically immunosuppressive blood level". means a blood level at which pimecrolimus shows evidence of causing general systemic immunosuppressive effects, such as suppression of transplant or tissue rejection or characteristic immunosuppressant adverse side effects, including but not limited to, opportunistic infections, such as those produced by bacterial, viral or fungal pathogens or lymphoproliferative disorders, such as lymphomas or other malignancies. This level could readily be determined in an individual patient by one of skill in the art by means of routine clinical assessment.

### Treatment of asthma with pimecrolimus

In studies involving both the mouse and rat models of asthma pimecrolimus has been shown to inhibit the allergen induced airway eosinophilia and IL-4 cytokine production in both single and multiple challenge models.

Having regard to the anti-inflammatory activity of pimecrolimus and its influence on airways hyper-reactivity, most particularly its specific effects in mitigating, reversing or preventing inflammation of the airway epithelium of the lung, pimecrolimus is useful for the treatment, in particular prophylactic treatment of obstructive or inflammatory airways disease. Tnus by continued and regular administration over prolonged periods of time pimecrolimus is useful in providing advance protection against recurrence of bronchoconstrictor or other symptomatic attack consequential to obstructive or inflammatory airways disease or for the control, amelioration or reversal of basal status of such disease.

Having regard to the anti-inflammatory activity of pimecrolimus on the epithelia of the gastrointestinal tract, it is also useful for treating IBD (e.g., UC and CD).

In treating inflammatory disease, the objective is to prevent or hinder the progression of the inflammatory processes, as well as to reverse existing inflammation, thus the words "treatment" and "treating" as used throughout the present specification are to be understood as embracing both prophylactic and symptomatic modes of therapy.

Obstructive or inflammatory airways diseases to which the present invention applies include asthma, pneumoconiosis, chronic obstructive airways or pulmonary disease (COAD or COPD), adult respiratory distress syndrome (ARDS), and allergen-induced inflammation of the airways, including seasonal allergic rhinitis and allergic asthma, as well as exacerbation of airways hyper-reactivity consequent to other drug therapy, e.g., aspirin or beta-agonist therapy.

The present invention is applicable to the treatment of asthma of whatever type or genesis, including intrinsic and, especially, extrinsic asthma. It is applicable to the treatment of allergic (atopic/IgE-mediated) asthma. It is also applicable to the treatment of non-atopic asthma, including, e.g., bronchitic, exercise induced and occupational asthma, asthma induced following bacterial infection and other non-allergic asthmas. It is further applicable to the treatment of wheezy infant syndrome (infant, incipient asthma).

The invention is applicable to the treatment of pneumoconiosis of whatever type or genesis including, e.g., aluminosis, anthracosis, asbestosis, chalicosis, ptilosis, siderosis, silicosis, tobacoosis and byssinosis.

The invention is applicable to the treatment of COPD or COAD including chronic bronchitis, pulmonary emphysaema or dyspnea associated therewith.

The invention is also applicable to the treatment of bronchitis of whatever type or genesis including, e.g., acute, arachidic, catarrhal, chronic, aoupus or phthinoid bronchitis etc.

Having regard to its anti-inflammatory activity, in particular in relation to inhibition of eosinophil activation, pimecrolimus is also useful for the treatment of eosinophil-related disorders, e.g., eosinophilia, in particular eosinophil-related disorders of the airways, e.g., involving morbid eosinophilic infiltration of pulmonary tissues, including hypereosinophilia as it effects the airways and/or lungs, as well as, for example, eosinophil-related disorders of the airways consequential or concomitant to Loffier's syndrome, eosinophilic pneumonia, parasitic, in particular metazoal infestation, including tropical eosinophilia, bronchopulmonary aspergillosis, polyarteritis nodosa, including Churg-Strauss syndrome, eosinophilic granuloma and eosinophil-related disorders affecting the airways occasioned by drug reaction.

Having regard to its ability to interact synergistically with immunosuppressive and/or anti-inflammatory, drug substances, pimecrolimus is also useful as co-therapeutic agents for use in conjunction with such drugs, e.g., as potentiators of therapeutic activity of such drugs or as means of reducing required dosaging or potential side effects of such drugs. Drug substances with which pimecrolimus may suitably be co-administered for treatment of inflammatory conditions of the airways include drugs as set forth in Table 1, for example, gtucocorticoids and corticosteroids, non-steroidal anti-inflammatory agents and bronchodilators. Drug substances with which pimecrolimus may suitably be co-administered for treatment of IBD include drugs as set forth in Table 3, in particular, corticosteroids. Diseases to which such co-therapy may be applied include, e.g., any disease or condition requiring anti-inflammatory drug therapy, e.g., as hereinbefore set forth. In particular, pimecrolimus are suitable for use in co-therapy as aforesaid, e.g., for the purposes of anti-inflammatory or anti-asthmatic treatment as set forth in A, e.g., to achieve steroid-sparing effect.

Therefore, in accordance with the foregoing the present invention provides:
A. Methods for treatment of EIDs, e.g., as hereinbefore set forth, particularly disorders characterized by inflammation of the airways epithelia or the epithelia of the GI tract, in a subject in need thereof, comprising administering to said subject an effective amount of pimecrolimus, e.g.,

A method of treating airways hyperreactivity; of effecting bronchodilation; of treating obstructive or inflammatory airways disease; or of treating IBD, including UC or CD; in a subject in need thereof, which method comprises administering to said subject an effective amount of pimecrolimus.

The methods of the invention, e.g., as defined under A, may further comprise coadministration with one or more drugs, for example as set forth in Tables 1 or 3. For example, pimecrolimus may be used in combination with a corticosteroid in a patient receiving corticosteroid treatment to reduce the level of corticosteroid administration.

The present invention also provides for the use of pimecrolimus in the preparation of a medicament for any method or in the treatment of any disease or condition as hereinbefore set forth, e.g., as defined under A above, for example, use of pimecrolimus in the preparation of a medicament for treatment of EIDs, e.g., disorders characterized by inflammation of the airways epithelia or the epithelia of the GI tract, for example, use of pimecrolimus in the preparation of a medicament for treating airways hyperreactivity; for effecting bronchodilation; for treating obstructive or inflammatory airways disease; or for treating IBD, including UC or CD.

In addition, this invention provides for the use of pimecrolimus in the manufacture of a medicament for administration to a patient in an amount which is effective to treat an EID but which is not systemically immunosuppressive, e.g., for treatment of a disease or condition as described above.

The present invention further provides pharmaceutical compositions comprising pimecrolimus for use in a method or in the treatment of a disease or condition as hereinbefore set forth, e.g., as defined under A above, for example, pharmaceutical compositions comprising pimecrolimus for treatment of EIDs, e.g., disorders characterized by inflammation of the airways epithelia or the epithelia of the gastrointestinal tract, for example, pharmaceutical compositions comprising pimecrolimus for treating airways hyperreactivity; for effecting bronchodilation; for treating obstructive or inflammatory airways disease such as asthma; or for treating IBD, including UC or CD.

In addition, the present invention provides pharmaceutical compositions comprising pimecrolimus for administration to a patient in an amount which is effective to treat the EID but is not systemically immunosuppresive, e.g., for treatment of a disease or condition as described above.

### Pimecrolimus

Pimecrolimus (also called ASM981 and ELIDEL®) is an ascomycin macrolactam-derivative with anti-inflammatory properties, chemical name is 33-epichloro-33-desoxy-ascomycin. Pimecrolimus is an ascomycin macrolactam-derivative developed originally for topical and oral treatment of inflammatory skin diseases such as atopic dermatitis and psoriasis. Pimecrolimus binds to macrophilin-12 and inhibits the Ca-dependent phosphatase calcineurin. As a consequence, it inhibits T cell activation and blocks the transcription and release of both Th1 and Th2 inflammatory cytokines. Pimecrolimus exhibits a high level of anti-inflammatory activity in animal models of skin inflammation after both topical and systemic application. In contrast to CyA and FK506, pimecrolimus has only a low potential to impair systemic immune responses as shown in rats in the graft-versus-host reaction, the antibody formation to sheep red blood cells and kidney transplantation (Figure 7), indicating an increased therapeutic window of pimecrolimus regarding its anti-inflammatory versus immunosuppressive activity. Tachyphylaxis has not been observed.

Elimination of pimecrolimus is predominantly via metabolism involving an oxidative mechanism catalyzed by the CYP3A enzyme subfamily; consequently there is potential for drug-drug interactions with compounds that are metabolized by this family. Repeated oral administration does not lead to drug accumulation in the blood or tissues, nor to the induction of drug-metabolizing enzymes in the liver. pimecrolimus is well-tolerated in man after oral dosing up to 60 mg (or 30 mg b.i.d.) over 28 days.

More recently topical pimecrolimus, in the form of 1% cream, has been used with great success in the treatment of psoriasis, atopic dermatitis and contact dermatitis. In studies oral treatment in patients with moderate to severe plaque psoriasis also proved highly successful. Pimecrolimus belongs to the macrolatam derivative family and as with other members of this family it binds to macrophilin 12 and inhibits calcineurin. Pimecrolimus is able to block the activation of both T cells and mast cells and the synthesis and release of inflammatory cytokines. Pimecrolimus and combinations and oral formulations are disclosed in U.S. Patent Nos. 6,197,781 and 6,004,973 and in EP 0 427 680 B1, WO 01/90110 A1 and WO 97/03654, all of which are hereby incorporated by reference for all purposes.

*Structure of "33-epichloro-33-desoxy-ascomycin" (pimecrolimus)* 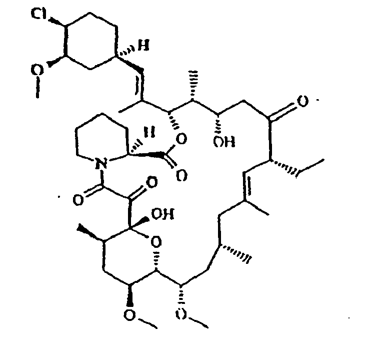 Pimecrolimus has been successfully tested in animal models and in clinical trials against inflammatory skin diseases using a topical formulation.

### Example 1

### Gene expression study of pimecrolimus in psoriasis patients

A clinical study was initiated with an oral formulation of pimecrolimus to treat psoriatic patients. Pimecrolimus was evaluated as treatment for psoriasis using an oral dose of 60 mg (30 mg b.i.d) during a 4-week clinical study. The cohort included 10 patients; 8 received the treatment and 2 received a placebo. Blood samples were collected before the first administration to establish the gene expression baseline and on days 13 or 14 after treatment with pimecrolimus or placebo and submitted to gene expression analysis in order to gain insights into the mechanisms of action of the compound. The clinical data demonstrate a reduction of psoriasis in the 8 pimecrolimus-treated patients.

### Pimecrolimus oral formulation

The oral formulation of pimecrolimus used in this study was formulated as shown below. The "solid dispersion" formulation of pimecrolimus consists of pimecrolimus (20%), Poloxamer188 (10%) and HPMC (70%) and the manufacture of this formulation is as disclosed in U.S. Patents Nos. 6,004,973 and 6,197,781 both of which are hereby incorporated by reference in their entirety and for all purposes. In addition, pimecrolimus and combinations and oral formulations are disclosed in U.S. Patent Nos. 6,197,781 and 6,004,973 and in EP 0 427 680 B1, WO 01190110 A1 and WO 97103654, all of which are hereby incorporated by reference for all purposes.

Pimecrolimus is a macrolide which occurs in two pseudopolymorphic forms, a hydrate (form A) and an anhydrous form (form B). Both forms can be used to prepare the solid dispersion product, in the oral formulation below the hydrate (form A) is used. In a preferred embodiment, the anhydrous form of pimecrolimus (form B) is used as disclosed in WO 99/01458, which is hereby incorporated by reference herein in its entirety.

The oral formulation used in this study were 20 mg tablets consisting of:

| **Component** | **mg/dosis** |
|---|---|
| Pimecrolimus solid dispersion 20% | 100.0 |
| Lactose, anhydrous | 98.75 |
| Polyvinylpolypyrrolidon XL | 50.0 |
| Magnesium stearate | 1.25 |
| Total tablet weight | 250.0 |

Placebo tablets matching the 20 mg active tablets were formulated as follows:

| **Component** | **mg/dosis** |
|---|---|
| Hydroxypropylmethyl-cellutose 3 CPS (Shin-Etsu) | 45.0 |
| Poloxamer 188 | 5.0 |
| Lactose, anhydrous | 148.75 |
| Polyvinylpolypyrrolidon XL | 50.0 |
| Magnesiumstearate | 1.25 |
| Total tablet weight | 250.0 |

The dose used in the study was 60 mg/day, given as 30 mg b.i.d. To make this dose the 20 mg tablets were cut in half using a special cutter (Tablettenteiler NR.96) so as to obtain 30 mg of pimecrolimus or a matched amount of placebo.

### Pharmacokinetics of pimecrolimus

In this study involving multiple oral administrations of various doses of pimecrolimus to human patients, it was found that both the maximum blood level (Cₘₐₓ) and the area under the curve of blood level over time (AUCτ) (AUC may be measured over the specific dosing interval or over a 24-hour period) were dose proportional (see Table 4 below). Mean Cₘₐₓ and AUCτ for the 20 mg q.d. and 20 mg b.i.d. dose groups were comparable. Assuming AUCτ is identical for both the morning and evening doses in the 30 mg b.i.d. dose group, mean AUC at steady-state over 24 hours is calculated to be about 590 ng•h/mL on day 13 and 562 ng•h/mL on day 28.

**Table 4. Summary of Cₘₐₓ and AUCτ Following Multiple Oral Administrations of Pimecrolimus to Psoriatic Patients for 28 Days**

| | C_{max,b} (ng/mL) Mean ± SD | | | AUC_{τ,b} (ng·h/mL) Mean ± SD | | |
|---|---|---|---|---|---|---|
| Dose group | Day 1 | Day 13 | Day 28 | Day 1 | Day 13 | Day 28 |
| 5 mg q.d. | 8.7 ± 2.3 | 9.7 ± 2.5 | 9.9 ± 4.8 | 23.3 ± 13.0 | 44.5 ± 18.2 | 35.5 ± 15.8 |
| 10 mg q.d. | 25.9 ± 8.5 | 21.8 ± 7.9 | 24 ± 9.2 | 80.6 ± 31.4 | 102.8 ± 41.4 | 90.9 ± 35.4 |
| 20 mg q.d. | 42.4 ± 10.8 | 55.3 ± 21.4 | 58.3 ± 25.4 | 172.6 ± 56.0 | 253.0 ± 40.1 | 257.7 ± 65.9 |
| 20 mg b.i.d. | 41.3 ± 16 | 54.7 ± 36.3 | 41.7 ± 16.5 | 139.9 ± 39.2 | 259.6 ± 122.6 | 229.5 ± 90.9 |
| 30 mg b.i.d. | 49.1 ± 21.6 | 54.5 ± 27.0 | 41.1 ± 8.4 | 205.8 ± 64.0 | 294.9 ± 60.1 | 281.0 ± 32.8 |

In this study pimecrolimus was rapidly absorbed with a median tₘₐₓ across all the dose groups and collection days of between 0.8 and 2.0 hours. The time to steady-state (as assessed from trough concentrations) was between about 6 and 13 days which is consistent with the long terminal half-life of 50-100 hours recorded after the final dose. There was no effect of age or weight on the apparent clearance of pimecrolimus.

In this study an AUC₍₀₋₂₄₎ of about 400 ng·h/mL and greater was associated with a significant clinical response in the target epithelial tissue. The AUC over 24 hours measured on day 13 for the 30 mg b.i.d. dose of pimecrolimus was 590 ng·h/mL and this was associated with a highly significant clinical improvement and minimal side effects or signs of systemic immunosuppression and no evidence of such adverse side effects as opportunistic infections, such as those produced by bacterial, viral or fungal pathogens or lymphoproliferative disorders, such as lymphomas or other malignancies.

In this study the relationship of trough concentration to clinical results was also determined. Trough concentrations of about 7 ng/mL and above were associated with a significant clinical response in the target epithelial tissue. However, even the highest median blood trough concentration reached during the study, I.e., 18 ng/ml on day 21 of the 30 mg BID dose schedule, did not result in any significant side effects or signs of systemic immunosuppression such as opportunistic infections, such as those produced by bacterial, viral or fungal pathogens or lymphoproliferative disorders, such as lymphomas or other malignancies.

At the dose levels, Cₘₐₓ and AUC shown in Table 4 there were no serious adverse side effects in any patient in the study. Specifically, no clinically significant change was observed, at any dose, Cₘₐₓ or AUC level, in; physical examination, blood pressure, electrocardiogram, safety laboratory tests (serum chemistries) or renal function including serum BUN and creatinine levels and there was no evidence of general systemic immunosuppression or the characteristic adverse side effects of immunosuppressant drugs, i.e., opportunistic infections, such as those produced by bacterial, viral or fungal pathogens or lymphoproliferative disorders, such as lymphomas or other malignancies.

In this specification, the terms "blood level" and "serum level" are used interchangeably and both mean the level of a drug as routinely measured by standard laboratory practice in the patients blood sample after separation of the red and white blood cells for convenience in the laboratory determination.

### Gene expression study

The RNA from blood samples drawn from each patient in the study was extracted and tested on individual DNA microarrays, thus allowing, for each patient, a comparison of gene expression before and after pimecrolimus treatment. The main purpose was to establish a profile of gene modulation to help in the understanding of the clinical effect of pimecrolimus in inflammatory conditions in general and the drugs potential side effects. Genes that were consistently modulated were sorted in functional categories.

### Gene expression profiles

In recent years, advances in several technologies have made it possible to monitor the expression level of a large number of genetic transcripts within a cell at any one time, this is referred to as "gene expression profiling". In organisms for which the complete genome is known, it is possible to analyze the transcripts of all genes within the cell. With other organisms, such as humans, for which there is an increasing knowledge of the genome, it is possible to simultaneously monitor large numbers of the genes within the cell (see, e.g., Schena et al., Science, Vol. 270, pp. 467-470 (1995); Lockhart et al., Nature Biotech., Vol. 14, pp. 1675-1680 (1996); Blanchard et al., Nature Biotech., Vol. 14, p. 1649 (1996); Ashby et al.,.U.S. Patent No. 5,569,588, issued October 29, 1996)) and proteins (see, e.g., McCormack et al., Anal. Chem., Vol. 69, pp. 767-776 (1997); Chait-BT, Nature Biotech., Vol. 14, p. 1544 (1996)).

Applications of this technology have included, for example, identification of genes which are up- or down-regulated in various physiological states, particularly diseased states. Additional uses for transcript arrays have included the analyses of members of signaling pathways, and the identification of targets for various drugs. Such applications are based upon the knowledge the abundances of cellular constituents such as mRNA species within a cell, change in response to virtually any alteration in a cell's biological state or environment. Such alterations include specific drug treatments or regimens.

Thus, a measurement of these gene expression profiles provides simultaneous information about the translational activity of large numbers of genes whose expression may be unregulated or down-regulated by exposure to a particular drug. Therefore these profiles contain a wealth of information about the nature and effect of the specific drug and, in addition, such a gene expression profile would be uniquely determined by the specifics of the treatment with a particular drug.

The gene expression profiling performed in connection with a study of the effect of pimecrolimus produced a consistent genomic profile of pimecrolimus (about 160 genes). The compound was shown to down-regulate the expression of genes belonging to the Macrolactam target pathway (macrophilin 12), cellular activation and proliferation (Histone2, Histone 3.3, cyclin D2) among blood cells, and to strongly down regulate the expression of inflammatory mediators (Leukotriene A4 hydrolase, prostaglandin endoperoxide synthase). Pimecrolimus efficacy as clinically observed may also be due to the dramatic down-regulation of genes necessary for chemotaxis and cellular migration at the site of inflammation, including LFA-1, P-selectin ligand and L-selectin.

Of special interest in this study, was the finding that, in addition to the general powerful anti-inflammatory effect of pimecrolimus, several genes, known to be involved in asthma and IBD pathophisiology, were specifically and significantly down-regulated by the administration of pimecrolimus. The asthma related genes included:
- RANTES, expressed in eosinophils from atopic asthmatics (see Raychaudhuri et al., Acta. Derm. Venereol., Vol. 79, No. 1, pp. 9-11 (1999)) and present in lung inflammation and asthma (see Chihara et al., J. Allergy Clin. Immunol., Vol. 100, No. 6, Part 2, pp. S52-S55 (1997)), Alam et al., Am. J. Respir. Crit. Care Med., Vol. 153, No. 4, Part 1, pp. 1398-1404 (1996)). In addition, RANTES antagonism has been shown to block hyperreactivity and eosinophil recruitment in a mouse model of asthma (see Gonzalo et al., J. Exp. Med., Vol. 188, No. 1, pp. 157-167 (1998)).
- GALECTIN-3, up-regulated in asthma (see Monteseirin et al., J. Exp. Med., Vol. 183, No. 6, pp. 2571-2579 (1996)).
   Thromboxane A2 receptor, causes bronchoconstriction in asthma (see Shen et al., J. Biomed. Sci., Vol. 5, No. 3, pp. 153-172 (1998)).
   Glutathione peroxidase, elevated with eosinophil count in the blood of asthmatics (see Misso et al., J. Leukoc. Biol., Vol. 63, No. 1, pp. 124-130 (1998)).

These findings strongly suggest that pimecrolimus would be a highly effective treatment for asthma as well as other inflammatory diseases including IBD. The genomic analysis of the peripheral blood from the patients before and after treatment showed a consistent genomic signature of pimecrolimus. The data showed that pimecrolimus acts via multiple targets and has a powerful and broad based anti-inflammatory activity. Many of the markers identified by expression profiling following pimeaolimus treatment indicate that pimecrolimus would be a highly effective treatment for asthma and IBD.

The development of inflammation requires the migration of cells to the site and pimecrolimus impairs multiple steps of the migration as chemoattraction, leukocytes rolling, firm adhesion to the endothelium and extravazation.

The down-regulation of chemoattractant RANTES would tend to prevent new inflammatory cells from reaching the site of inflammation. The down-regulation of RANTES expression is of particular interest since it perfectly matches the reported *in vitro* effect of pimecrolimus namely, impaired inflammatory mediators release, decrease of the IgE promoter activity, decrease of T cell activation and proliferation (see Bochelen et al., J. Pharmacol. Exp. Ther., Vol. 288, No. 2, pp. 653-659 (1999)).

L-selectin and P-selectin ligand, key molecules in the leukocyte rolling steps are also down-regulated. P-selectin ligand can bind various lectins and participate in celvendothelium interactions. This protein is post-transcriptionally modified and one of the modification variants is known as cutaneous lymphocyte antigen (CLA). CLA is thought to be responsible for targeting lymphocytes to the skin or other epithelial tissue. The down-regulation of CLA would also impair lymphocyte migration at the site of extravazation.

Pimecrotimus also down regulates the synthesis of cytoskeleton proteins, thus impairing cellular mobility. In addition, major enzymes such as prostaglandin endoperoxide synthase and leukotriene A4 hydrolase are down regulated. Kallistafin, an inhibitor of kinin generation is up-regulated. The action of pimecrolimus thus seems to efficiently block the inflammatory potential of circulating cells.

Taken together data showed that pimecrolimus is able to down-regulate:
- The macrophilin regulation pathway
- Cellular activation and proliferation
- HLA expression
- Cellular migration at the inflammation site
- Inflammation

No obvious toxic effect could be observed in the expression profile. No gene linked to a possible adverse effect or to a toxicity pathway was found to be regulated. The combination of the strong impact on RANTES, adhesion molecules and inflammatory proteins, such as Thromboxane A2 receptor (responsible for bronchoconstriction in asthma) indicates that pimecrolimus would be effective in the treatment of asthma.

Direct evidence on the efficacy on pimecrolimus in asthma is provided by studies showing the effect on lung eosinophilia rats. One study looked at the inhibitory effect of pimecrolimus on the aeroallergen induced eosinophil influx into the lungs of sensitized mice. This study found that pimecrolimus was able to reduce the eosinophil count in the lung and the eosinophil peroxidase activity after oral administration.

Another study looked at the effects of oral administration of pimecrolimus on antigen-induced pulmonary eosinophilia in actively sensitized Brown Norway Rats. This study found that pimecrolimus was effective when given orally as a microemulsion or dissolved in Neoral® placebo.

In addition to the finding that pimecrolimus down-regulates numerous genes known to be involved in the pathophysiology of asthma, an unexpected discovery of a previously unknown property of pimecrolimus was made during the clinical trials of this drug. This discovery concerns a property of pimecrolimus that suggests that this drug would be a uniquely effective treatment for asthma and EIDs in general, such as IBD and should be able to substitute for part or all of the glucocorticoids or corticosteroids or immunosuppressants in a asthma or IBD treatment regimen and therefore substantially reduce the side effects from the use of these drugs to control severe or chronic asthma or IBD. This discovery is the marked preference of pimecrolimus for exerting its anti-inflammafory action in epithelial tissue in contrast to general systemic effects. In the studies of pimecrolimus in psoriasis it was shown that the drug has a skin selective anti-inflammatory effect and that it has preferential affinity for the skin and epithelial tissue in general due to its unique molecular characteristics, including but not limited to, its lipophilicity.

Pimecrolimus is highly active in animal models of skin inflammation after both topical and systemic administration. Pimecrolimus, when applied topically, permeates less through human (and animal) skin than, for example, FK506 and corticosteroids by factors of 9 and 60-120, respectively, indicating a lower potential to enter the systemic circulation and thus to exert systemic side effects. As is well-known, glucocorticoids are non-selective agents and are therefore associated with many systemic actions and a wide variety of side effects, whether applied topically or systemically. This is in contrast to pimecrolimus.

An example of the unexpected epithelial selectivity of pimecrolimus is shown in Figure 7 which shows a comparison of pimecrolimus to CyA and FK506 in the mouse model of ACD and in a series of animal models of systemic immunosuppression, such as kidney transplantation, graft vs. host reaction and antibody production. In the top left panel, oral pimecrolimus is more than 2-fold more efficacious than oral CyA in ACD and (bottom left) superior to FK506. However, pimecrolimus shows minimal general systemic immunosuppressive effects as compared to either CyA (top right) or FK506 (bottom right) in a series of transplantation and vaccination models.

Comparative studies in rats have shown, that pimecrolimus (q.d., subcutaneous (s.c.)) has a clearly increased therapeutic window of anti-inflammatory activity in the skin versus systemic immunosuppression as compared to other agents, such as FK506. Since 2 of the 3 models of systemic immunosuppression included s.c. application (= mimicking 100% transdermal delivery), these results are relevant for topical application as well.

Another study of tissue distribution in rats after single and multiple oral doses of pimecrolimus and FK506 found a higher affinity of pimecrolimus for skin than to any other organ in the body as compared with FK506.

In another study designed to explore the differences between pimecrolimus and FK506 and to determine whether these differences might be associated with a different tissue distribution, the exposure to pimecrolimus and FK506 in various organs after oral administration were measured. Animals received 2 oral doses of 25 mg/kg in an interval of 2 hours. Concentrations were measured by HPLC-MS in different tissues (including cutis, subcutis, blood, kidney, lymph nodes) 2, 6 and 24 hours after the last dosing and the AUC - time curve was calculated. Pimecrolimus was found consistently to have a higher skinlblood concentration ratio than FK506 (2 hours: 7.3 vs. 2.8; 6 hours: 13 vs. 1.6; 24 hours: 36 vs. 6.0). The AUC₀₋₂₄ₕ in skin calculated for pimecrolimus was twice as high as with FK506. In all other tissues, including blood, lymph nodes and kidney, exposure to pimecrolimus was lower than to FK506.

In conclusion, these data suggest that pimecrotimus has a higher affinity for skin than FK506, and this finding is in line with the observed skin-selective pharmacological profile of pimecrolimus (see, Stuetz et al., Sem. Cutan. Med. Surg., Vol. 20, pp. 233-241 (2001)).

The resulting increased therapeutic window has been confirmed also in a recent study comparing the effects of pimecrolimus and FK506 on skin inflammation and lymph nodes during sensitization phase and elicitation phase of ACD, showing that pimecrolimus primarily interferes with inflammation in the skin, while FK506 acts primarily on the subcutaneous lymphoid tissue (see, Meingassner et al, J. Invest. Dermatol., in press (2002)).

In line with the pharmacological observations, distribution studies (oral application) in rats have shown that pimecrolimus has a higher affinity to skin and a lower affinity to lymph nodes than FK506. These distribution characteristics are relevant not only for oral, but also for topical application, as the compound enters systemic circulation after permeation through the skin.

Taken together, these data demonstrate that pimecrolimus has a skin selective distribution pattern and significantly greater anti-inflammatory activity in the skin after topical or oral application, with clear differences as compared to other immunosuppressants, for example, FK506.

Of great importance, for the treatment of EIDs in general, such as asthma and IBD, is the finding that the preferential action of pimecrolimus for epithelial tissue of skin also applies for other epithelial tissues, such the epithelial tissue lining the airways in the lung and the epithelial tissue or mucosa lining the GI tract. This was an unexpected finding and could only have been made during the clinical studies of pimecrolimus.

This finding suggests a remarkable property of pimecrolimus and means that administration of pimecrolimus, either systemically or topically, i.e., by inhalation in asthma or by enema or suppositories in IBD, will result in a significantly greater anti-inflammatory effect in the epithelial tissue of the lung or the GI tract as compared to general systemic anti-inflammatory effect or systemic side effects. This differential action will result in the same type of increased therapeutic window of anti-inflammatory activity in the lung for the treatment of asthma or in the GI tract in the treatment of IBD as pimecrolimus has clearly demonstrated in the treatment of psoriasis.

Thus, a given dose of the drug, given either orally or by inhalation or rectally, will produce an effective anti-inflammatory effect in the epithelial tissue of the lung or GI tract but much less general systemic effect or systemic side effects. This would allow the use of higher doses of pimecrolimus in patients with ElDs, such as asthma or IBD before the dose levels are limited by systemic side effects. Thus, pimecrolimus should be as effective or more effective than glucocorticoids or other immunosuppressants for the treatment of ElDs, such as asthma or IBD while having lower overall side effects.

In addition, pimecrolimus should be able to substitute for all, or at least some, of the otherwise necessary glucocorticoids or immunosuppressants in an established glucocorticiod and/or immunosuppressants requiring treatment regimen for an EID, such as IBD or asthma and therefore allow lower doses of glucocorticiod and/or immunosuppressants to be used with equal or even greater efficacy. This so-called "steroid sparing effect" or perhaps "immunosuppressant sparing effect" would be of such great value for minimizing the adverse effects of glucocorticiod or immunosuppressant therapy.

Thus, the large panel of inflammatory related genes that are down-regulated and the preferential action in epithelial and related tissue demonstrates that pimecrolimus would also be a uniquely effective treatment for any other disease in which an inflammatory process occurring in epithelial tissue is the cause of symptoms or morbidity or in which the inflammation of epithelial or related tissues, such as mucosa or mucus membranes, plays a prominent role in the pathology or pathogenesis of the disease, I.e., EIDs.

This novel and unexpected property of pimecrolimus would produce a hitherto unrecognized advantage in the use of pimecrolimus in the treatment of such "EIDs" by producing a "therapeutic window" effect in which a significant therapeutic effect could be achieved in inflamed epithelial or related tissues while producing minimal systemic effects, including adverse side effects. This therapeutic advantage of pimecrolimus would be in comparison to producing an equivalent therapeutic effect in a given EID by the administration of any other anti-inflammatory or immunosuppressant agents which does not posses the epithelial tissue preference of pimecrolimus. This would include, but not be limited to, corticosteroids, other immunosuppressants, such as CyA or FK506.

### Example 2

### Pimecrolimus treatment of animal model of IBD

Pimecrolimus administered p.o. is effective in the treatment of psoriasis in man as discussed above. Psoriasis and IBD, namely CD and UC, share common underlying pathological mechanisms which involve activation and expansion of disease-causing T cells of the Th1 class. This provided the rationale for the study of the existing oral form of pimecrolimus in a unique mouse model for IBD.

As discussed above, the "epithelium selective" aspect of the actions of pimecrolimus had been shown by the observation that pimecrolimus exhibits high anti-inflammatory activity in animal models of skin inflammation, but in contrast to CyA and FK506, it has only a low potential for systemic immunosuppression.

A well-established animal model for IBD exists (see Leach et al., Am. J. Pathol., Vol. 148, No. 5, pp. 1503-1515 (1996), and Powrie et al., Int. Immunol., Vol. 5, No. 11, pp. 1461-1471 (1993)). It is known that the transfer of syngeneic, naive T lymphocytes (CD4⁺CD45RB^{HI}) into SCID mice results in a severe colitis and wasting disease, SCID-IBD. Histopathologically, features of both CD and UC are represented in this model which has been used throughout the 1990s to bring the understanding of the pathoetiology of this disease of immune dysregulation in man to its present status. There is no other animal model of IBD which is considered relevant for the human disease, both in mechanism and in validity of clinical treatment. To date, this model has been used to show that blocking antibodies to TNF-α, IL-12 and IFN-γ have partial and transient protective effects. More recently, beneficial effects have also been reported with antibodies to CD134 ligand (OX40L), to CD154 and to β7 integrin or MAdCAM-1. Both CyA and dexamethasone treatment inhibit the colitis in this model but at doses associated with serious toxic effects. In addition, CyA treatment is associated with a general reduction in lymphocyte number, reflecting its potent immunosuppressant activity in this model.

In this study, SCID mice reconstituted with CD4⁺CD45RB^{HI} T lymphocytes were used. These mice are known to develop chronic colitis with characteristics similar to human IBD, as described above. Pimecrolimus is an immunomodulator with proven efficacy in animal models of inflammatory skin disease and in patients with atopic dermatitis or psoriasis while lacking the systemic immunosuppressive effects associated with FK506 or CyA, as discussed above. The intention of this study was to evaluate the effect of pimecrolimus in the SCID-IBD model and also to compare with CyA in order to evaluate the "epithelium selective" actions of pimecrolimus in this disease.

In this study, SCID mice were reconstituted with 2 x 10⁵ CD4⁺CD45RB^{HI} T cells and then treated with pimecrolimus (30, 60 and 100 mg/kg/day) or vehicle daily p.o. for 4 weeks. For comparison purposes another group of mice received CyA (15, 30 and 60 mg/kg/day) s.c. via osmotic mini-pump. BW was monitored throughout. On termination, blood was withdrawn, spleen, mesenteric lymph nodes (MLN) and colon were removed. Serum haptoglobin levels were measured (ELISA), colitis was assessed histologically and lymphocyte and neutrophil numbers were calculated from FACS analysis of blood, spleen and MLN cell suspensions.

The results of this study showed that control SCID-IBD mice in 3 studies (n=24 mice), lost 15-18% of the original BW 4 weeks post-T cell transfer. Pimecrolimus-treated (30, 60 or 100 mg/kg/day) mice lost 7.1, 8.8 or 3.2% but CyA treatment caused even the non-transferred SCID mice to lose BW.

Therefore, in this mouse model, oral administration of pimecrolimus for 4 weeks immediately following transfer of the disease-causing T cells, prevented the severe loss of BW associated with the disease in a dose-responsive manner over the dose range of 30-100 mg/kg/day. In comparison, CyA dose dependently (60, 30 and 15 mg/kg/day via mini pump) further worsened the loss of BW (see Figures1 and 2).

In order to determine the efficacy of ASM981 treatment in preventing intestinal mucosal damage in SCID-IBD mice, a histological examination of the colon sections was performed according to defined morphological parameters. All colon sections were assessed according to a scheme awarding degrees of severity governed by the extent of cell infiltration and inflammation with a score of 8 being the maximum.

The severe colitis observed in the placebo-treated SCID-IBD mice was significantly inhibited by ASM981 treatment. The mice treated with ASM981 were reproducibly found to have only mildly inflamed colons at the dose of 100 mg/kglday while treatment with 60 and 30 mg/kg/day resulted in a moderate and moderate to severe inflammation, respectively. Treatment with a high dose of 60 mg/kg/day CyA was also effective and resulted in a mild inflammation score, whereas treatment with 30 mg/kg/day resulted in a mild to moderate inflammation. Doses lower than 30 mg/kg/day failed to significantly inhibit colitis (see Figure 3).

Dexamethasone treatment also inhibited the colitis but the doses needed (3 and 10 mg/kg/day p.o.) were associated with steroid-related accelerated loss in BW. In addition, both CyA and dexamethasone strongly inhibited lymphocyte numbers in blood, spleen and MLN, an effect which was not observed with pimecrolimus indicating that pimecrolimus at 100 mg/kg/day, while highly effective in inhibiting ongoing colitis, does not have the general immunosuppressive properties of CyA and dexamethasone.

Pimecrolimus treatment also improved in a dose-dependent fashion the diarrhea which is characteristic for severe colitis. Approximately half of the mice in the 100 mg/kg/day group had macroscopically normal feces. In summary, pimecrolimus treatment of SCID-IBD mice reduced the severity of colitis in a dose-dependent fashion. In contrast, CyA was effective only at high doses that caused toxic systemic side effects (see Figure 3 - SCID mice were reconstituted with 2 x 10⁵ CD4⁺CD45RB^{HI} T cells and treatment started one day later. Pimecrolimus was given p.o. daily and CyA (given as SANDIMMUN® ) was given via osmotic mini-pump. All studies were terminated after 29 days when a piece of colon was washed and fixed in a formalin solution, paraffin embedded, cut into 3 µm sections, stained with H&E and scored on a scale of 0 (no effect) - 8 (severe). The maximum score was 8 and non-transferred mice scored as 0. Box plots represent the median, range and SD of the individual scores (N=7 or 8) for each group.)

Systemic inflammation, as measured by an acute phase response protein (serum haptoglobin levels), and as neutrophilia in blood and spleen, was strongly inhibited by pimecrolimus treatment.

Haptoglobin production, which is elevated in SCID-IBD mice, was significantly reduced in pimecrolimus-treated mice by 68-92% over the dose range 30-100 mg/kg/day, similarly blood and spleen neutrophilia were reduced by 85-96% and by 74-83%, respectively. CyA treatment elicited similar levels of inhibition but with significance only at the highest doses of 30 and 60 mg/kg/day (see Figures 4 and 5).

Neutrophil infiltration into the colon is a feature of both IBD in man and of the SCID-IBD model. Measurement of the myeloperoxidase activity in colon homogenates is correlated to the numbers of neutrophils in the colon. Using this technique it was found that the neutrophil infiltration into the colon was completely blocked at 100 mg/kg/day pimecrolimus and by 72% at 60 mg/kg/day (see Figure 6). CyA treatment also significantly reduced MPO activity in the inflamed colons. At doses of 30, 15 and 7.5 mg/kg/day, MPO activity was inhibited by 79.8, 66.5 and 30.2%, respectively (see Figure 6).

The clear difference between the systemic effects and selective therapeutic effects of pimecrolimus treatment is shown by the fact that in these studies pimecrolimus, at concentrations of up to 100 mg/kg/day, did not significantly affect lymphocyte numbers in blood or spleen but significant reduction in lymphocyte numbers was measured in MLN. In contrast, CyA-treated mice at doses of 60, 30 and 15 mg/kg had reduced lymphocyte counts in blood, spleen and MLN (see Figures 8 and 9). This result suggests that pimecrolimus at therapeutic doses does not have the powerful general systemic immunosuppressive effects, and side effects, of CyA.

These results show that pimecrolimus does not have the general immunosuppressive activities of CyA due to selective *in vivo* distribution of the compound to epithelial tissues, in this case to the mucosa of the GI tract.

### Delayed treatment results

In order to determine if pimecrolimus is also effective once IBD is established, SCID mice were treated with pimecrolimus at a dose level of 100 mg/kg/day at days 7, 14 or 21 following the transfer of the disease causing CD4⁺ T cells. The study was terminated after 41 days, i.e., the mice were treated for 33, 26 and 19 days, respectively. The results are shown in Figures 10-12, and described below.

Figure 10 shows that pimecrolimus is highly-effective in reversing the loss of BW associated with established/ongoing mild to severe colitis. SCID mice were reconstituted with 2 x 10⁵ CD4⁺CD45RB^{HI} T cells and treatment with pimecrolimus (100 mg/kg/day p.o.) started after 7, 14 or 21 days and continued daily until 41 days. Treatment with the placebo formulation started after 7 days. BW was monitored once a week for the first 2 weeks and then twice weekly. Data are expressed as mean percent BW relative to the weight on day 0 ± s.e.m. for n = 9 (PBS), n = 8 (ASM(7d) & ASM(21d) and n = 7 (ASM(14d) and placebo) mice per group. PBS refers to the non-transferred mice which have been pooled with regard to percent BW values since no treatment dependent changes were found. p<0.01 relative to the placebo-treated group at 41 days.

Figure 11 shows that pimecrolimus inhibits the ongoing acute phase response in SCID mice with established IBD, as shown by the inhibition of haptoglobin. SCID mice were reconstituted with 2 x 10⁵ CD4⁺CD45RB^{HI} T cells and treatment with pimecrolimus (100 mg/kg/day p.o.) started after 7, 14 or 21 days and continued daily until 41 days. Treatment with the placebo formulation started after 7 days. On day 41, mice were killed and serum kept for haptoglobin assay. Data are expressed as mean haptoglobin (mg/mL) ± s.e.m. for n = 9 (PBS), n = 8 (ASM(7d)) and n = 7 (ASM(14d), ASM(21d) and placebo) mice per group. PBS refers to the non-transferred mice which have been pooled with regard to haptoglobin values since no treatment dependent changes were found. p<0.01 relative to the placebo-treated group at 41 days.

Figure 12 shows that pimecrolimus strongly inhibits blood and spleen neutrophilia associated with established/ongoing colitis. SCID mice were reconstituted with 2 x 10⁵ CD4⁺CD45RB^{HI} T cells and treatment with pimecrolimus (100 mg/kg/day p.o.) started after7, 14 or 21 days and continued daily until 41 days. Treatment with the placebo formulation started after 7 days. On day 41, mice were killed, blood and spleens removed, cell suspensions prepared and incubated with relevant antibodies to identify the neutrophil population by FACS analysis. Data are expressed as the mean neutrophil numbers, calculated from the percent positive cells and the total cell number per sample, ± s.e.m. for n mice (n in each bar) per group. p<0.05 calculated relative to the placebo-treated group.

The overall conclusion of this study was that pimecrolimus administration, dose dependently, reduced the disease in SCID-IBD mice without suppressing the proliferative function of the circulating lymphocytes, in contrast to CyA, this finding was consistent with the epithelium selective action of pimecrolimus seen in the psoriasis trials. The potent and selective anti-inflammatory action of pimecrolimus indicates great and previously unrecognized therapeutic potential for pimecrolimus in IBD (see Moore et al., "Pimecrolimus Possesses Significant IBD Inhibiting Actions in a SCID Mouse Model", presented at the11th International Congress of Mucosal Immunology, June 16-20,2002, Orlando, FL, USA).

### The clinical assessment of asthma

As part of the methods of this invention, the clinical status and extent of symptoms may be assessed according to the protocol outlined below.

### Measurement of asthma severity

The clinical of the severity of asthma in any given patient involves an objective determination of the nature and severity of each episode, as well as consideration of the number and frequency of episodes of asthma. The clinician relies on a number of criteria to asses the severity of any given episode of attach of asthma. This assessment is well-known to clinicians and follows the steps outlined below. This assessment protocol is from, Cecil Textbook of Medicine, 21^{st} Edition, Goldman and Bennett, Eds., W.B. Saunders Company, Philadelphia, PA, Chapter 74 (2000).

### Physical and laboratory examination

On physical examination the clinician will note many of the common features of acute asthma attacks including a rapid respiratory rate (often 25-40 breaths per minute), tachycardia, and pulsus paradoxus (an exaggerated inspiratory fall in the systolic pressure). The magnitude of the pulsus is related to the severity of the attack.

Inspection of the patients thoracic region may reveal that the patient is using their accessory muscles of ventilation. If this is occurring the skin over the thorax may be retracted into the intercostal spaces during inspiration. This is a indication of a severe asthma attack. In addition, the chest will be seen to be hyperinflated, and the expiratory phase will be prolonged relative to the inspiratory phase. Percussion of the thorax demonstrates hyperresonance, with loss of the normal variation in dullness due to diaphragmatic movement. Auscultation of the chest will reveal wheezing, this is the cardinal physical finding in asthma. Wheezing, commonly louder during expiration but heard during inspiration as well, is characterized as polyphonic in that more than one pitch may be heard simultaneously. Accompanying adventitious sounds may indude rhonchi, which are suggestive of free secretions in the airway lumen, or rales, which are indicative of localized infection or heart failure. The loss of intensity or the absence of breath sounds in a patient with asthma is an indication of severe airflow obstruction and therefore a severe episode.

The assessment of the severity of an asthma episode will also include an evaluation by the clinician of laboratory tests and perhaps the most important of these tests will be the results of a battery of pulmonary function tests. The interpretation of these test is simplified by a basic understanding that a decrease in airflow rates throughout the vital capacity is the cardinal pulmonary function abnormality during an asthmatic episode. The peak expiratory flow rate (PEFR), the forced expiratory volume in the first second (FEV₁) and the maximal mid-expiratory flow rate (MMEFR) are all decreased in asthma and the degree of change from normal (for that patient) is the most accurate measure of severity.

In very severe asthma, dyspnea may be so severe as to prevent the patient from performing a complete spirogram. In this case, if 2 seconds of forced expiration can be recorded, useful values for PEFR and FEY, can be obtained. The assessment of attack severity, as shown in Table 5, must be assessed by objective measures of airflow. As the attack resolves, the PEFR and the FEV₁ increase toward normal in concert while the MMEFR remains substantially depressed; as the attack resolves further, the FEV₁ and the PEFR may normalize while the MMEFR remains depressed. Even when the attack has resolved clinically, residual depression of the MMEFR is not uncommon; this depression may resolve over a prolonged course of treatment. If the patient is able to cooperate such that more complete measurements of lung function can be made, lung volume measurements will demonstrate an increase in both total lung capacity (TLC) and residual volume (RV); the changes in TLC and RV resolve with treatment.

**Table 5. Relative Severity of an Asthmatic Attack as Indicated by PEFR, FEV₁ and MMEFR**

| **Test** | **% of Predicted Value** | **Asthma Severity** |
|---|---|---|
| PEFR | ≥ 80% | No spirometric abnormalities |
| FEV₁ | ≥ 80% | |
| MMEFR | ≥ 80% | |
| PEFR | ≥ 80% | Mild asthma |
| FEV₁ | ≥ 70% | |
| MMEFR | 55-75% | |
| PEFR | ≥ 60% | Moderate asthma |
| FEV₁ | 45-70% | |
| MMEFR | 30-50% | |
| PEFR | <50% | Severe asthma |
| FEV₁ | <50% | |
| MMEFR | 10-30% | |

| | | |
|---|---|---|
| PEFR = peak expiratory flow rate MMEFR = maximal mid-expiratory flow rate FEV₁ = forced expiratory volume in the first second. | | |

### Arterial blood gases

In cases of severe asthma a determination of arterial blood gases may provide some additional indication of severity. Blood gas analysis need not be undertaken in individuals with mild asthma. If the asthma is of sufficient severity then blood gas analysis is indicated and will probably show hypoxemia and hypocarbia. With the subject breathing room air, the PaCO₂ is usually between 55 and 70 mm Hg and the PaCO₂ between 25 and 35 mm Hg. At the onset of the attack, an appropriate pure respiratory alkalemia is usually evident; with attacks of prolonged duration, the pH normalizes as a result of a compensatory metabolic acidemia. A normal PaCO₂ in a patient with moderate to severe airflow obstruction is reason for concern, as it may indicate that the mechanical load on the respiratory system is greater than can be sustained by the ventilatory muscles and that respiratory failure is imminent.

When the PaCO₂ rises in such settings, the pH falls quickly, because the bicarbonate stores have become depleted as a result of renal compensation for the prolonged preceding respiratory alkalemia. Because this chain of events can take place rapidly, close observation is indicated for asthmatics with "normal" PaCO₂ levels and moderate to severe airflow obstruction.

Other laboratory findings are of lesser value in determining severity of a particular episode however they may be useful in the assessment of more chronic conditions. Asthmatic subjects are frequently atopic; thus, blood eosinophilia is common. In addition, elevated serum levels of IgE are often documented and may serve as a measure of chronicity since epidemiologic studies indicate that asthma is unusual in subjects with low IgE levels. Alone the same lines specific radioallergosorbent tests (RASTs), which measure IgE directed against specific offending antigens, can be conducted. In rare instances during severe asthma attacks, serum concentrations of aminotransferases, lactate dehydrogenases, muscle creatine kinase, ornithine transcarbamylase, and anti-diuretic hormone may be elevated.

The chest radiograph of a subject with asthma is often normal and therefore of little use in the assessment of severity. However, severe asthma is associated with hyperinflation, as indicated by depression of the diaphragm and abnotmally lucent lung fields and this can be determined from a chest radiograph. In addition, complications of severe asthma, including pneumomediastinum or pneumothorax, may be detected radiographically.

The electrocardiogram, save for sinus tachycardia, is also usually normal in acute asthma.. However, right axis deviation, right bundle branch block, "*P pulmonale*" or even ST-T wave abnormalities may arise during severe asthma and resolve as the attack resolves and these finding can also be used in determining severity.

Asthma is easy to recognize in a young patient without co-morbid medical conditions who has exacerbating and remitting airway obstruction accompanied by blood eosinophilia. A rapid response to bronchodilator treatment is usually all that is needed to establish the diagnosis. The degree of severity can be assessed by use of the above techniques but by far the most important method is to perform pulmonary function test the interpretation of which is given in Table 1.

### Pharmaceutical compositions

The pharmaceutical compositions of the invention are preferably administered as compositions containing the therapeutic agent in combination with one or more pharmaceutically acceptable carriers. The compositions may be administered alone or in combination with at least one other agent, such as stabilizing compound, which may be administered in any sterile, biocompatible pharmaceutical carrier, including, but not limited to, saline, buffered saline, dextrose and water. The compositions may be administered to a patient alone or in combination with other agents, drugs or hormones.

The pharmaceutical compositions of this invention may be administered by an number of routes including, but not limited to, oral, inhalation of an aerosol or powder form, i.v., intramuscular (i.m.), intra-articular, intra-arterial, intramedullary, intrathecal, intraventricular, transdermal, s.c., intraperitoneal, intranasal, enteral, topical, sublingual or rectal means.

In addition to the active ingredient, these pharmaceutical compositions may contain suitable pharmaceutically acceptable carriers comprising excipients and auxiliaries which facilitate processing of the active compounds into preparations which can be used pharmaceutically. In a preferred embodiment, excipients include anhydrous lactose, polyvinylpolypyrrolidon XL and magnesium stearate. Further details on techniques for formulation and administration may be found in the latest edition of Remington's "Pharmaceutical Sciences', Maack Publishing Co., Easton, PA.

Pharmaceutical compositions for oral administration can be formulated using pharmaceutically acceptable carriers well-known in the art in dosages suitable for oral administration. Such carriers enable the pharmaceutical compositions to be formulated as tablets, pills, aerosol, dry powders, dragees, capsules, liquids, gels, syrups, slurries, suspensions and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores.

The preferred oral formulation of pimecrolimus used in the methods of this invention may be formulated as shown above. The "solid dispersion" formulation of pimecrolimus consists of pimecrolimus (20%), Poloxamer188 (10%) and HPMC (70%) and the manufacture of this formulation is as disclosed in U.S. Patent Nos. 6,004,973 and 6,197,781, both of which are hereby incorporated by reference in their entirety and for all purposes. In addition, pimecrolimus and combinations and oral formulations are disclosed in U.S. Patent Nos. 6,197,781 and 6,004,973 and in EP 0 427 680 B1, WO 01/90110 A1 and WO 97/03654, all of which are hereby incorporated by reference for all purposes.

Furthermore, pimecrolimus is a macrolide which occurs in two pseudopolymorphic forms, a hydrate (form A) and an anhydrous form (form B). Both forms can be used to prepare the solid dispersion product, in the oral formulation disclosed above the hydrate (form A) is used. In one preferred embodiment, the anhydrous form of pimecrolimus (form B) is used as disclosed in WO 99/01458, which is hereby incorporated by reference herein in its entirety.

For topical or nasal administration, penetrants appropriate to the particular barrier to be permeated are used in the formulation. Such penetrants are generally known in the art. Pimecrolimus may also be formulated as a aerosol for inhalation administration using techniques known in the art. The pharmaceutical compositions of the present invention may be manufactured in a manner that is known in the art, e.g., by means of conventional mixing, dissolving, granulating, dragee-making, levigating, emulsifying, encapsulating, entrapping or lyophilizing processes. After pharmaceutical compositions have been prepared, they can be placed in an appropriate container and labeled for treatment of an indicated condition. Such labeling would include amount, frequency, and method of administration.

### II-2 reporter gene assay

This assay method is disclosed and enabled in U.S. Patent No. 5,897,990, which is hereby incorporated by reference in its entirety and for all purposes. In order to measure the amount of systemic immunosuppresent effect or the concentration of a specific immunosuppressant, such as pimecrolimus, CyA, FK506 or corticosteroids that are present in the body fluids of a patient during treatment use is made of assay methods for monitoring the activity in bodily fluids of immunosuppressants affecting gene expression, including but not limited to, ascomycins, such as pimecrolimus; using a reporter gene assay, e.g., an IL-2 reporter gene assay for immunosuppressive cyclosporins, corticosteroids and ascomycins, or a c-jun reporter gene assay for immunosuppressive rapamycins.

In the reporter gene assay used in the methods of this invention, a chimeric gene construct is created comprising a reporter gene under the transcriptional control of a human promoter gene for a cytokine influenced by the immunosuppressant, e.g., pimecrolimus, CyA, FK-506, AZA or corticosteroids to be monitored, and this construct is incorporated into a suitable cell line. The cell line is preferentially a human T-cell or macrophage cell line, e.g., a Jurkat cell line, preferably selected for high native production of the desired gene product, for example, producing IL-2 in response to stimulation in the case of an IL-2 reporter gene assay. The reporter gene is any gene expressing a readily observable product, e.g., an enzyme marker such as β-galactosidase or luciferase, or any other suitable enzyme marker. For example, where the enzyme marker is β-galactosidase, the reporter gene is, e.g., a bacterial lacZ gene; where the enzyme marker is luciferase, the gene is, e.g., a Phorinus luciferase gene. Another suitable marker is the gene for the green fluorescent protein produced by the bioluminescent jellyfish, e.g., as described in Chalfie et al., Science, Vol. 263, pp. 802-805 (1994). The promoter gene is a promoter gene for a cytokine or protein for which transcription is stimulated or suppressed by the drug to be monitored.

For example, where the immunosuppressant effect to be monitored is produced by pimecrolimus, CyA, FK-506, AZA or corticosteroids, the promoter gene can be a promoter gene for mammalian IL-2, TGF-β, IFN-γ or IL-4, as it has been found that these drugs inhibit gene expression of these cytokines in mammals. Preferably, where the immunosuppressant effect to be monitored is produced by pimecrolimus, CyA, FK-506, AZA or corticosteroids, the promoter gene is the promoter for human IL-2.

Blood samples from the patient are diluted, their effect on gene expression in the above described system is measured, and the assay is standardized using known concentrations of the immunosuppressive material. The inhibition of gene expression is dose-dependent at low concentrations, and pimecrolimus, for example, can be measured at concentrations of less than 0.01 ng/mL using this system. In this assay the concentration required for 50% inhibition (the IC₅₀ value) for pimecrolimus is 1.5 nM as compared to the IC₅₀ value for FK506 which is 1.0 nM. Thus, this IL-2 receptor assay technique can be used to measure and compare general systemic levels of immunosuppression produced by a variety of drugs and the concentrations of specific immunosuppressants including but not limited to pimecrolimus, CyA, FK506, AZA or corticosteroids.

In a further aspect of this assay method, where a patient is receiving a combination of immunosuppressant drugs, the reporter gene assay method can be targeted to a particular immunosuppressant by removal or inhibition of other immunosuppressants using specific affinity separation techniques, e.g., utilizing specific antibodies (polyclonal or monoclonal, preferably monoclonal) or specific receptors to such immunosuppressants (e.g., cyclophilin for CyA, FKBP-12 for FK-506 or rapamycin, or specific receptors for corticosteroids) and then measuring the remaining immunosuppressant. In particular, when patients are treated with combinations of corticosteroids and IL-2 gene suppressing immunosuppressant drugs, such as pimecrolimus or FK506 or CyA, it is desirable first to remove the corticosteroids.

For example, where a patient, e.g., an asthma or IBD patient is receiving pimecrolimus and in addition, a corticosteroid, e.g., prednisone, the pimecrolimus level can be measured by taking blood from the patient, contacting the blood with beads coated with monoclonal antibody for prednisone, and then separating the blood from the beads, measuring the suppression of IL-2 gene expression in an IL-2 reporter gene assay in the presence of the blood, and comparing the level of suppression to that observed in the presence of known concentrations of pimecrolimus. Other drugs which may be used in combination with cyclosporins or ascomycins, e.g., AZA, brequinar, desoxyspergualine, and rapamycins, do not affect the IL-2 reporter gene assay at therapeutic, e.g., low nanomolar, concentrations, and therefore need not be removed.

Ascomycins, including pimecrolimus and rapamycins, bind to macrophilins in the cells (e.g., FK-binding protein or FKBP-12), and cyclosporins bind to cyclophilins. These compounds may be freed from their respective immunophilins by treatment with an excess of a nbn-immunosuppressive, immunophilin-binding competitor or by denaturing the immunophilin, e.g., by heat-treatment. Examples of non-immunosuppressive, cyclophilin-binding cyclosporins suitable for use as binding competitors for immunosuppressive cyclosporins are described in EPA 484,281. Examples of non-immunosuppressive, macrophilin-binding macrolides suitable for use as binding competitors for immunosuppressive macrolides, such as rapamycins and ascomycins are described in WO 94118207, both of which are incorporated by reference herein in their entirety.

In addition to the IL-2 reporter gene assay, the concentration of immunosuppressants, such as pimecrolimus and FK506 can be measured by the mixed-lymphocyte reaction (see Meo, The MLR in the Mouse", Immunological Methods, Lefkovits and Pemis, Eds., p. 227 Academic Press, NY). In the MLR assay, the IC₅₀ value for pimecrolimus is 9.0 nM while that for FK506 is 1.0 nM, a nine-fold difference.

### REFERENCES CITED

All references cited herein are incorporated herein by reference in their entirety and for all purposes to the same extent as if each individual publication or patent or patent application was specifically and individually indicated to be incorporated by reference in its entirety for all purposes. The discussion of references herein is intended merely to summarize the assertions made by their authors and no admission is made that any reference constitutes prior art. Applicants reserve the right to challenge the accuracy and pertinence of the cited references.

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatus within the scope of the invention, in addition to those enumerated herein, will be apparent to those skilled in the art from the foregoing description and accompanying drawings. Such modifications and variations are intended to fall within the scope of the appended claims. The present invention is to be limited only by the terms of the appended claims, along with the full scope of equivalents to which such claims are entitled.

## Claims

1. Use of pimecrolimus for the manufacture of a medicament for the treatment of epithelial inflammatory disorder (EID) in a human, the amount of pimecrolimus being sufficient to ameliorate at least one symptom of the EID while minimizing systemic immunosuppression, wherein the systemic immunosuppression is determined by measuring the expression profile of at least one gene selected from the group consisting of macrophilin 12, histone2, histone 3.3, cyclin D2, and combinations thereof; leukotriene A4 hydrolase, prostaglandin endoperoxide synthase, and combinations thereof; LFA-1, P-selectin ligand, L-selectin, and combinations thereof; and RANTES, GALECTIN-3, thromboxane A2, glutathione peroxidase, and combinations thereof; and measuring the effect of pimecrolimus.

2. The use of claim 1, wherein the epithelial inflammatory disorder (EID) is asthma.

3. The use of claim 1, wherein the epithelial inflammatory disorder (EID) is inflammatory bowel disorder (IBD).

4. The use of claim 1, wherein the systemic immunosuppression is determined by an IL-2 reporter gene assay.

5. The use of claim 1, wherein the pimecrolimus is administered orally as a tablet, a capsule or a liquid suspension or solution or by intravenous infusion, transdermal delivery, inhalation of an aerosol or rectally by enema or suppository.

6. The use of claim 1, further comprising measuring the blood level of pimecrolimus.

7. The use of claim 6, wherein the blood level of pimecrolimus is less than that required to produce systemic immunosuppression.

8. The use of claim 7, wherein the blood level of pimecrolimus is less than the IC₅₀ of the IL-2 reporter gene assay.

9. The use of claim 7, wherein the blood level of pimecrolimus is less than 2.0 ng/mL.

10. The use of claim 7, wherein the blood level of pimecrolimus is less than 1.5 ng/mL.

11. The use of claim 7, wherein the blood level of pimecrolimus is less than 1.0 ng/mL.

12. The use of claim 7, wherein the blood level of pimecrolimus is less than 0.75 ng/mL.

13. The use of claim 7, wherein the blood level of pimecrolimus is less than 0.5 ng/mL.

14. The use of claim 7, wherein the blood level of pimecrolimus is less than 0.25 ng/mL.

15. The use of claim 6, wherein the blood level of pimecrolimus is less than that required to produce a mean steady-state area under the curve (AUC) over 24 hours of 1000 ng•h/mL.

16. The use of claim 15, wherein the blood level of pimecrolimus is less than that required to produce a mean steady-state AUC over 24 hours of 600 ng•h/mL.

17. The use of claim 15, wherein the blood level of pimecrolimus is less than that required to produce a mean steady-state AUC over 24 hours of 400 ng•h/mL.

18. The use of claim 15, wherein the blood level of pimecrolimus is less than that required to produce a mean steady-state AUC over 24 hours of 200 ng•h/mL.

19. The use of claim 6, wherein the blood level of pimecrolimus has a trough level in a steady-state condition of greater than 7 ng/mL but less than 20 ng/mL.

20. The use of claim 19, wherein the trough level is less than 15 ng/mL.

21. The use of claim 19, wherein the trough level is less than 10 ng/mL.

22. The use of claim 6, wherein the blood level in a steady-state condition has a Cₘₐₓ is less than 70 ng/mL.

23. The use of claim 22, wherein the Cₘₐₓ is less than 60 ng/mL.

24. The use of claim 22, wherein the Cₘₐₓ is less than 50 ng/mL.

25. The use of claim 22, wherein the Cₘₐₓ is less than 40 ng/mL.
